(19) Europäisches Patentamt
European Patent Office
Office européen des brevets

(11) **EP 3 344 744 B1**

(12) **EUROPÄISCHE PATENTSCHRIFT**

(45) Veröffentlichungstag und Bekanntmachung des Hinweises auf die Patenterteilung:
**24.06.2020  Patentblatt 2020/26**

(21) Anmeldenummer: **16760406.5**

(22) Anmeldetag: **19.08.2016**

(51) Int Cl.:
*C12M 1/107* *(2006.01)*         *B01D 19/02* *(2006.01)*
*C12M 1/02* *(2006.01)*          *C12M 1/21* *(2006.01)*

(86) Internationale Anmeldenummer:
**PCT/EP2016/069722**

(87) Internationale Veröffentlichungsnummer:
**WO 2017/036824 (09.03.2017 Gazette 2017/10)**

(54) **VORRICHTUNG ZUR VERRINGERUNG DER SCHAUMBILDUNG IN EINEM BIOGASFERMENTER**

DEVICE FOR REDUCING FOAMING IN A BIOGAS FERMENTER

DISPOSITIF SERVANT À RÉDUIRE LA FORMATION DE MOUSSE DANS UN FERMENTEUR DESTINÉ À LA PRODUCTION DE BIOGAZ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **31.08.2015  DE 102015114510**

(43) Veröffentlichungstag der Anmeldung:
**11.07.2018  Patentblatt 2018/28**

(73) Patentinhaber: **Agraferm GmbH**
**85276 Pfaffenhofen (DE)**

(72) Erfinder:
• **KUBE, Jürgen**
  **GU29LF Guildford (GB)**
• **STERNEKIEKER, Mirko**
  **15345 Altlandsberg (DE)**
• **HARDERS, Dirk**
  **24107 Kiel (DE)**
• **ROSSBERG, Andreas**
  **39443 Straßfurt (DE)**
• **LEHMANN, Tommy**
  **37355 Gerterode (DE)**

(74) Vertreter: **HGF Europe LLP**
**Neumarkter Str. 18**
**81673 München (DE)**

(56) Entgegenhaltungen:
EP-A1- 1 179 584      DD-A1- 60 022
DE-A1- 10 224 172     DE-A1- 19 850 822
DE-C- 871 438         DE-C- 972 899
GB-A- 1 075 100

**Beschreibung**

Die Erfindung betrifft einen Fermenter mit einer Vorrichtung zur Verringerung der Schaumbildung und/oder Verbesserung der Entgasung vom Gärmedium in einem Biogasfermenter. Des Weiteren betrifft die vorliegende Erfindung ein Verfahren zur Verringerung und Verhinderung der Schaumproduktion und/oder Verbesserung der Entgasung vom Gärmedium in einem Biogasfermenter mittels einer Vibrationsvorrichtung.

In Biogasanlagen werden organische Substrate zu Biogas, einer Mischung von Methan, $CO_2$ und Spurengasen umgesetzt. Bei diesem Prozess bilden sich in der Flüssigphase kleine Gasblasen; die Gasblasen koaleszieren und verlassen den Fermentationsbrei aufgrund der Dichtedifferenz nach oben. Oftmals enthält der Gärbrei noch suspendierte Feststoffe, die das Entgasungsverhalten behindern. Als Folge steigt der Volumenanteil des Gases in der Fermentationsbrühe. In der Literatur hat sich für diesen Volumenanteil der englische Fachbegriff Gas Hold-Up $\varepsilon_G$ durchgesetzt. Der Gas Hold-Up ist definiert als

$$\varepsilon_G = \frac{V_G}{V_G + V_L}$$

Dabei ist

$\varepsilon_G$     [-] Gas Hold-Up
$V_G$     [m$^3$] Gasvolumen in der Fermentationsbrühe
$V_L$     [m$^3$] Flüssigkeitsvolumen in der Fermentationsbrühe

Steigt der Gas Hold-Up in einer Biogasanlage unkontrolliert an, kann dies erst zum Verstopfen der Gasleitungen führen und anschließend sogar zur Beschädigung des Fermenterdachs bzw. des auf dem Biogasreaktor montierten Gasspeichers. Ein unkontrolliertes Ansteigen des Gas Hold-Ups wird als Schaum bezeichnet.

Schaum auf Biogasanlagen kann grob in zwei Kategorien eingeteilt werden. Als "Blasendispersion" werden Schäume bezeichnet, bei dem der Gas Hold-Up kleiner 50% ist, in der Regel zwischen 10 und 20%. Die Blasendispersion zeichnet sich dadurch aus, dass die Gasblasen ungefähr gleichförmig im Fermenter verteilt sind. Blasendispersionen treten auf, wenn das Gärmedium eine hohe Viskosität aufweist und die Rührwerke im Fermenter ausfallen oder die Gasblasen nicht zum koaleszieren bringen können. Eine andere Form des Schaums ist der Polyederschaum, der sich in Tenside enthaltenden Flüssigkeiten bildet, wenn Gas eingebracht wird. Die tensidhaltige Flüssigkeit bildet ein dreidimensionales Netzwerk flüssiger Lamellen, welche das Gas einschließen und so Polyeder bilden. Der Polyederscchaum sammelt sich an der Oberfläche des Gärmediums an.

Der Polyederschaum wird häufig durch die Oberflächenspannung herabsetzende Substrate induziert. So sind Zuckerrüben, Zuckerrübenpressschnitzel, Getreide, Getreideganzpflanzensilage, Abfälle wie Speisereste, Fettabscheiderfette, Blut und Schlachtabfälle bekannt dafür, dass sie im Fermenter zu Schaumbildung führen können. Besonders schnellabbaubare Substrate führen in schlecht durchmischten Fermentern zu einer lokalen Versäuerung, d.h. die Hydrolyse und Acidogenese läuft kurzfristig schneller ab als die Methanbildung. Als Folge sammeln sich organische Säuren an, der pH-Wert sinkt und der Anteil Dissoziationsgrad der Säuren reduziert sich. Der niedrige pH-Wert und der gestiegene Anteil der undissoziierten Säuren hemmt die Methanogene weiter. Durch das lokale Absinken des pH-Werts wird zusätzlich $CO_2$ entgast und die Gasproduktion steigt kurzfristig und lokal stark an, was die Schaumbildung verstärkt.

Die Schaumbildung wird durch folgende Eigenschaften beeinflusst:

- Rheologie und Fließverhalten der Fluide
- Oberflächenspannung und Oberflächenenergie zwischen Gas-, Flüssig- und Festphase
- Leerrohrgeschwindigkeit des Gases im Fermenter
- Partikelgrößenverteilung und Blasengrößenverteilung
- Eingetragene Rührenergie, Scherratenverteilung im Fermenter
- Koaleszenzverhalten der Blasen

Zum jetzigen Zeitpunkt existiert noch kein mechanistisches Modell der Schaumbildung auf Biogasanlagen, welches bei der Vorhersage von Schaum-Ereignissen helfen könnte. Schaumbildung spielt aber auch abseits von Biogasanlagen eine wichtige Rolle. Schäume treten unter anderem in Destillationskolonnen von Erdölraffinerien, in Kläranlagen zur Behandlung von kommunalen und industriellen Abwässern und in Bioreaktoren der weißen Biotechnologie auf. In diesen Industriezweigen wurden Verfahren und Methoden entwickelt, um Schaum-Ereignisse zu bekämpfen.

Biogasanlagen unterschieden sich in einigen Punkten von den oben genannten Anwendungen. In Biogasanlagen findet die Gasbildung in der gesamten Flüssigphase statt. Die Flüssigphase wird also nicht von Gasen durchströmt wie in der Kläranlage. Auch findet die Blasenbildung nicht nur lokal an einer heißen Oberfläche statt wie in einer Destillationskolonne.

Das Gärmedium in einer Biogasanlage ist im Allgemeinen hoch viskos und zeigt nicht-Newtonsche Eigenschaften. Gärmedien mit geringem Feststoffanteil (etwa <10%) können als strukturviskose Fluide beschrieben werden. Fluide mit höherem Feststoffanteil weisen eine Fließgrenze auf und können als Bingham oder Casson-Fluide beschrieben werden. Allen passenden Flüssigkeitsmodellen ist gemein, dass sie bei geringen Scherraten eine hohe Viskosität aufweisen. Kleine Gasblasen, die nur eine geringe Auftriebsgeschwindigkeit aufweisen, erzeugen somit nur eine geringe Scherrate und sind von einer hochviskosen Flüssigkeit umgeben, die die Widerstandskräfte erhöht.

Anders als in Bioreaktoren und Kläranlagen werden in Fermenter auf Biogasanlagen nur sehr geringe Rühr-Leistungen eingetragen. Die übliche Volumen-dissiperte Leistung in Biogasreaktoren liegt bei ca. 2 bis 10 W/m$^3$. In Kläranlagen werden Volumen-dissiperte Leistungen von ca. 500 W/m$^3$, in Reaktoren zur Hochzelldichtefermentation von Mikroorganismen wie *E. coli* werden Werte von bis zu 2000 W/m$^3$ erreicht. Demzufolge sind die Schergradienten in Biogasanlagen deutlich geringer als in anderen Fermentationen.

Das Gärmedium von Biogasanlagen ist stark gepuffert. Beim Abbau von organischen Substraten entstehen neben Methan auch $CO_2$ und Ammoniak. Bei einem typischen pH-Wert im Biogasreaktor von 7 bis 8 stellt sich ein starker Ammonium-Carbonat-Puffer von ca. 0,2 mol/L $NH_4$-N bzw. 0,4 mol/L Hydrogencarbonat ein. Pro Liter Gärmedium sind also ca. 9 L $CO_2$ als Hydrogencarbonat gelöst. Methan hingegen löst sich kaum im Gärmedium, bei 40 °C lösen sich etwa 0,03 L $CH_4$ pro Liter Wasser. Im Mittel werden $CO_2$ und Methan gleich schnell gemäß der Stöchiometrie der Gärung gebildet. Durch den geringen Energieeintrag kommt es jedoch zu einer starken Übersättigung gelöster Gase, die sich jedoch bei beginnender Blasenbildung - z.B. bei lokalem Energieeintrag - schlagartig entgasen.

Die Geometrie von Biogasanlagen unterscheidet sich deutlich von andern Reaktionssystemen. Oftmals sind landwirtschaftliche Biogasreaktoren nur 6 bzw. 8 m hoch, da Betonbehälter in dieser Höhe am preiswertesten gestaltet werden können. Der Durchmesser solcher Fermenter beträgt bis zu 35 m. Industrielle Biogasanlagen können 20 m oder höher werden. Sie sind häufig schlanker ausgeführt und weisen ein Höhen zu Durchmesser-Verhältnis von ca. 1 auf. Biogasreaktoren gehören zu den größten bekannten Bioreaktoren mit typischen Fermentervolumen von 1000 bis 7000 m$^3$.

Der relative Gasdruck auf Biogasanlagen beträgt selten mehr als 5 mbar. Dieser Druck genügt, um das Gas bis zu den Verbrauchern zu drücken. Höhere Gasdrücke würden deutlich teurere Dachsysteme erfordern. Eine Vorrichtung zur Bekämpfung von Schaum darf daher keinen hohen Druckverlust aufweisen. Das bedeutet, dass durch die Anordnung und Verwendung einer Vorrichtung zur Schaumbekämpfung in dem Fermenter kein Druckabfall stattfinden darf. Dies könnte zur Folge haben, dass der Druck nicht mehr ausreichend ist um das Gas bis zu den Verbrauchern zu drücken.

In Bioreaktoren der weißen Biotechnologie ist der Fermenterdruck nur von untergeordneter Wichtigkeit, da diese Reaktoren meist autoklavierbar ausgeführt sind und somit Drücke von mindestens 2 bar absolut aushalten.

Eine Besonderheit von Biogasanlagen in Deutschland ist die unterschiedliche Anforderung des Genehmigungsrechts für kleine und große Anlagen. Kleine Anlagen können nach dem Baurecht genehmigt werden. Die Anlage muss nach den allgemein anerkannten Regeln der Technik gebaut werden und entsprechende Sicherheitstechnik aufweisen. Ab einer bestimmten Größe muss die Anlage nach dem Bundes-Immissionsschutzgesetz (BImschG) genehmigt werden. Dann muss die Anlage nach dem Stand der Technik ausgestattet sein und z.B. ein Störfallkonzept aufweisen. Stand der Technik bedeutet in diesem Zusammenhang "entwickeltes Stadium der technischen Möglichkeiten zu einem bestimmten Zeitpunkt, basierend auf entsprechenden gesicherten Erkenntnissen von Wissenschaft, Technik und Erfahrung" (DIN EN 45020:2006). Der Anlagenbetreiber muss nachweisen, dass die Anlage bei einem Störfall (z.B. einem Schaumereignis) automatisch einen eigensicheren Zustand annimmt. Dies wird üblicherweise durch hart-verdrahtete Schaltungen realisiert, die unabhängig von der Anlagensteuerung bei Auslösen von Sensoren automatisch gewisse Aggregate an- oder abschalten. Wird eine kleine Bestandsbiogasanlage erweitert, muss sie häufig noch dem BImschG nachgenehmigt werden, was ein umfangreiches Nachrüsten der Anlagensteuerung nach sich zieht. Andere Länder erfordern generell den Stand der Technik oder "Best Practice" bei der Ausführung der Biogasanlagen.

Ein Überblick über Schaumprobleme auf Biogasanlagen ist in dem Fokusheft "Schaumbildung in Biogasanlagen" 2015 des Deutschen Biomasse Forschungszentrums (DBFZ) zu finden. Das Fokusheft ist beim DBFZ erhältlich oder online abrufbar unter https://www.energetische-biomassenutzung.de/fileadmin/user_upload/Downloads/Verö6ffentlichungen-/FH_schaumbildung_web.pdf.

Um Schäden durch schäumende Gärmedien auf Biogasanlagen zu verhindern wurde ein Schaumtester entwickelt. Er wird in der DE 20 2013 000 693 U1 beschrieben. Der Schaumtester ermöglicht ein frühes Erkennen von schäumenden Substraten. Der Betreiber der Biogasanlage kann dann auf die Zugabe der schäumenden Substrate verzichten. Dies kann aber zu empfindlichen wirtschaftlichen Einbußen im Anlagenbetrieb führen.

Die einfachste Möglichkeit zur Schaumbekämpfung in Biogasanlagen ist die Zugabe von Pflanzenöl in den Reaktor. Dies kann manuell oder automatisiert über eine Schaumsonde und eine Dosierpumpe erfolgen. Schaumsonden sind meist kapazitive Sonden, die ein einfaches binäres Digitalsignal abgeben. Andere Ausführungsformen sind Schwinggabeln, Leitfähigkeitssonden oder Drehsonden.

Außer Pflanzenöl können auch spezielle Antischaummittel auf Kohlenstoff oder Silikonbasis verwendet werden. Letztere können in Biogasanlagen zu Siloxanen umgesetzt werden, die bei der Biogasverwertung in Blockheizkraftwerken zu Problemen führen können. Antischaummittel kosten ca. 5 € pro Liter. Die kontinuierliche Zugabe von Antischaummitteln stellt eine nicht unerhebliche finanzielle Belastung der Biogasanlage dar. Silikonhaltige Antischaummittel sind in der DE 19 850 822 B4 beschrieben.

In der weißen Biotechnologie werden sogenannte Schaumzentrifugen eingesetzt. Diese trennen ein schäumendes Gemisch in eine flüssige und eine gasförmige Phase und führen die Flüssigphase zurück in den Fermenter. Eine solche Schaumzentrifuge ist in der DE 2 548 441 A1 offenbart. Die Schaumtrennung wird außerhalb des Reaktors durchgeführt. Schaumzentrifugen haben einen sehr hohen Druckverlust, da Zweiphasengemische in Rohrleitungen deutlich höhere Druckverluste als einphasige Fluide erzeugen und können daher nicht ohne weiteres in Biogasanlagen eingesetzt werden. Zudem würde die Schaumzentrifuge bei einer Blasendispersion nicht gut funktionieren, da die hohe Viskosität des Gärmediums die Entmischung in der Zentrifuge erschwert. Gleiches gilt für mechanische Schaumzerschlagungsapparate, wie sie z.B. in der DD 60022 A1 beschrieben werden.

Ein einfaches Verfahren zur Zerstörung von Polyederschaum ist das Umpumpen und Sprühen von Reaktorflüssigkeit auf die Oberfläche des Biogasreaktors. Bei dünnen Medien wird dabei eine Rotationsströmung an der Oberfläche geschaffen und jeder Teil der Schaumdecke strömt an der Düse vorbei. Diese Lösung funktioniert gut bei Polyederschaum und niederviskosen Medien. Zur Bekämpfung von Blasendispersionen oder bei Fermentern mit großer Oberfläche funktioniert diese Lösung nur eingeschränkt. Verfahren zur Reduktion der Schaumschicht in einem Biogasreaktor sind in der US 4,334,997 A sowie der DE 4 402 56 A1 beschrieben.

Die EP 2 803 729 A2 offenbart ein Verfahren zur Vergärung von Tiermist. Bei hohen Trockenrückständen im Fermenter kommt es regelmäßig zu einem Anstieg des Gas Hold-Ups. Der Anstieg des Gas Hold-Ups wird dadurch bekämpft, indem ein Nebenrührwerk mit einer Reynolds-Zahl von mindestens 100 knapp unterhalb des Flüssigkeitsspiegels angeordnet wird. Das Nebenrührwerk erzwingt eine Koaleszenz der dispergierten Blasen und hat sich somit für die Schaumbekämpfung in Anlagen zur Vergärung von Tiermist bewährt. Allerdings zeigte in internen Versuchen der Einsatz eines solchen Nebenrührwerks auf einer Biogasanlage, die etwa zur Hälfte Zuckerrübenpressschnitzel und Hühnertrockenkot vergärt und großblasigen Polyederschaum aufweist, nicht den erwarteten Erfolg. Die Leistungsaufnahme des Nebenrührwerks ist mit 15 kW außerdem recht hoch.

Die EP 1 757 562 A1 beschreibt ein Verfahren zur Gärrestaufbereitung mittels eines Vibrationsscherkraftfilters (VSEP). In diesem Filter werden Vibrationen verwendet, um lokal hohe Scherraten zu erzeugen, die ein Verstopfen einer Membranoberfläche verhindern. Dieses Verfahren nutzt Vibrationen in Kombination mit den besonderen rheologischen Eigenschaften von aufbereitetem Gärmedium einer Biogasanlage.

Die DE 3 943 416 A1 beschreibt ein Verfahren zum Entwässern von Gülle, Abwässern und dünnflüssigen Schlämmen. Zur besseren Abtrennung von Feststoffen aus einer Flüssigkeit wird vorgeschlagen, die Flüssigkeit mit akustischen Vibrationen zu beaufschlagen.

Die DE 10 224 172 B4 beschreibt eine Vorrichtung und ein Verfahren zur Abtrennung von Sand aus einer Flüssigkeit. Zur Unterstützung der Sedimentation wird die Flüssigkeit mit Vibrationen beaufschlagt.

Die GB 1075100 A beschreibt ein Verfahren zur Entgasung von niederviskosen Flüssigkeiten und zur Zerstörung von feinem Schaum in Abwässern aus der Filmproduktion mittels Ultraschall. Die Flüssigkeit wird in eine Kammer geführt und dort mit Frequenzen von 10 bis 100 kHz beaufschlagt. Die maximale Viskosität beträgt 80 mPas.

Die DE 1 769 953 B offenbart eine Vorrichtung zur Ultraschall-Entgasung von Flüssigkeiten. Schäumende Flüssigkeiten werden darin durch eine Kammer geführt, in der eine Ultraschall-Vibrationsplatte im Winkel von 10 bis 30° zur Waagerechten angeordnet ist.

Die DE 871438 B beschreibt ein Verfahren zur Beseitigung von Schaumbildungen in flüssigen Stoffen, wobei die Schaummassen durch niedrige Frequenzen, welche von Luftschallfedern erzeugt werden, zerstört werden (siehe Anspruch 1). Auf Seite 2, Zeilen 4 bis 8 wird beschrieben, dass die Luftschallsender ihre Schallwellen entweder von oben auf die Schaummassen einwirken lassen oder dass die Sender innerhalb der Schaummassen angeordnet werden. Dadurch werden die Gasblasen der Schaummassen zum Platzen bzw. Zusammenbrechen gebracht (siehe Seite 2, Zeilen 14 und 15). Als mögliche Schallsender werden alle Arten von Luftschallsendern genannt, welche für große Leistung und niedrige Frequenz verwendet werden können: insbesondere Sirenen, Pfeifen oder elektrische Membransender (siehe Seite 2, Zeilen 18 bis 23). Hinsichtlich der Frequenz wird in den Zeilen 29 bis 35 der Seite 2 angegeben, dass diese auf 100 Hz abgestimmt ist und entweder kontinuierlich oder stoßweise erfolgen kann.

Die DE972899 B betrifft ein Verfahren und eine Vorrichtung zum Zerstören von Schaum in z. B. Gärbottichen sowie ein Verfahren, um die Entstehung von Schaum zu verhindern (siehe Seite 1, Zeilen 1 bis 5). Hierfür werden Kompressionswellen mittels einer mit breiten Flächen ausgestatteten Apparatur erzeugt, die an einem elektromagnetischen Rüttelgerät angebracht ist (siehe Seite 1, Zeilen 16 bis 20). Diese als Tellerapparatur bezeichnete Konstruktion, wird in die Schaummasse hineingesenkt, wodurch die Teller bzw. Ringe innerhalb des Schaums vibrieren können (siehe Seite 2, Zeilen 31 bis 36). In den Zeilen 81 bis 84 der Seite 2 wird offenbart, dass durch die Wahl verschieden hoher Abstände zwischen den Flüssigkeitsspiegel und dem Teller der schaumzerstörende Effekt je nach Bedarf gesteuert werden kann. Grundsätzlich soll die Wirkung aber durch Öffnen in den Teller erfolgen.

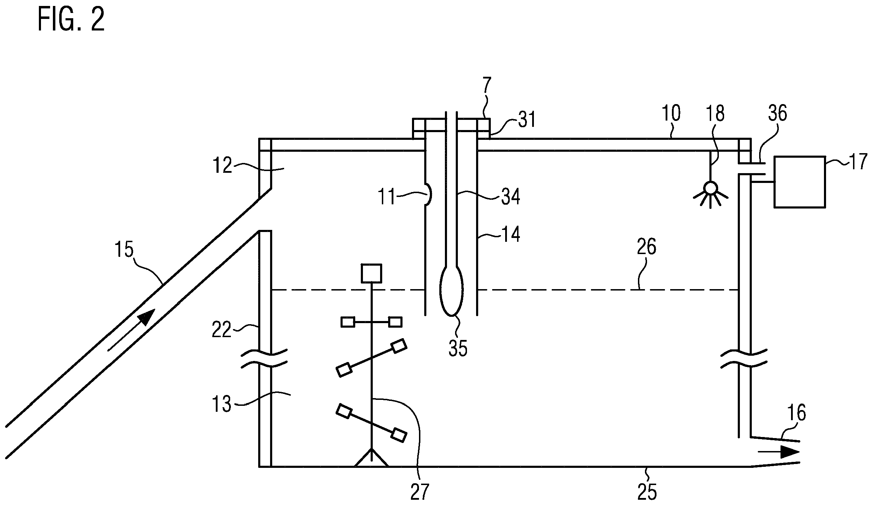

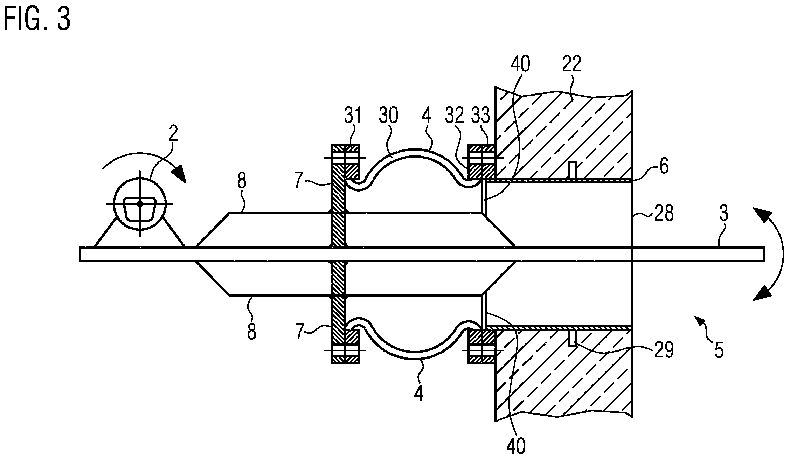

Die US 2006/0172405 A1 offenbart die Übertragung von Schwingungen auf Substrate für Biogas (siehe [00285]), um zelluläres Material aufzuschließen und dadurch eine erhöhte Biogasproduktion zu erzielen (siehe z.B. [0002] und [0013]). Der angegebene Frequenzbereich variiert je nach Anwendungsart von 1 kHz bis 10 kHz (siehe z.B. [0014], [0016], [0035], [0039] und [0044]) oder 1 kHz bis 2000 kHz (siehe z.B. [0044] und [0054]). Wie den Figuren 2 und 3 sowie den Absätzen [0062] und [0063] zu entnehmen ist, wird die Beschallung durch Kontaktplatten (Bezugszeichen 70), welche außen am Bioreaktor angebracht sind, auf das zelluläre Material nach innen übertragen. Es wird offenbart, dass die Beschallung entweder durch eine Vielzahl verschiedener Frequenzbereiche bereitgestellt wird, kontinuierlich übertragen wird oder unregelmäßig angesetzt wird. Am Ende desselbigen Absatz wird angegeben, dass ein bevorzugter Frequenzbereich zwischen 2 bis 7 kHz liegt. Diese Schrift scheint eher auf eine Disagreggation des aufzuschließenden Guts gerichtet zu sein.

Die DE 60130714 T2 offenbart Fermentationskulturen, bei welchen die Schaumbildung über einen Schaumdetektionssensor und ein Ultraschalloszillationshorn kontrolliert werden soll (siehe [0009]). Als Oszillationsfrequenz wird in [0022] der Frequenzbereich von 19-21 kHz angegeben.

Aus der Literatur und aus Praxisanwendungen sind Ultraschall-Sonden bekannt, die Biogasanlagen oder Faultürmen auf Kläranlagen eingesetzt werden, um mittels Ultraschall das Gärmedium aufzuschließen. Der Ultraschall bewirkt eine lokale Kavitation, die Fasern und Mikroorganismen zerstört. Solche Verfahren sind beispielsweise in der DE42 05 739 A1, DE 2013 225 322 A1, DE 10 2013 206 492 A1 und EP 2 769 764 A1 beschrieben. In der letztgenannten Schrift wird ein Leistungseintrag von ca. 2 kW in einen außerhalb des Fermenters liegenden Ultraschallreaktors erwähnt.

Eine Aufgabe der Erfindung ist es, einen Fermenter bereitzustellen, welche unkontrollierte Schaumproduktion in Biogasanlagen, verringert und/oder verhindert und/oder die Entgasung des Gärmediums verbessert.

Noch eine weitere Aufgabe der vorliegenden Erfindung besteht darin ein Verfahren bereitzustellen, um unkontrollierte Schaumproduktion in Biogasanlagen zu verringern und/oder zu bekämpfen und/oder die Entgasung des Gärmediums zu verbessern.

Durch die vorliegende Erfindung soll es ermöglicht werden, kostengünstig und einfach Biogasanlagen, insbesondere mit hohen Feststoffanteilen (>10%) mit Vorrichtungen zur Bekämpfung der Schaumproduktion auszustatten oder nachzurüsten.

Eine weitere Aufgabe der vorliegenden Erfindung liegt in der Bereitstellung eines Verfahrens zum Entgasen von Biogasreaktoren.

Die unabhängigen Ansprüche lösen eine oder mehrere der oben angegebenen Aufgaben. Vorteilhafte Ausgestaltungen der Erfindung sind in den jeweiligen Unteransprüchen angegeben.

Gemäß einem ersten Aspekt betrifft die Erfindung eine Vorrichtung zur Verringerung der Schaumbildung und/oder Verbesserung der Entgasung in einem Biogasfermenter dadurch gekennzeichnet, dass sie eine Vibrationsvorrichtung ist, welche zum Übertragen von Schwingungen auf ein im Biogasfermenter befindliches Gärmedium ausgebildet ist.

Die Schwingungen des Schalls führen lokal zu einem Austreiben von gelöstem $CO_2$ aus dem übersättigten Gärmedium und zum koaleszieren der reduzierten Gasblasen.

In einer bevorzugten Ausführungsform der Erfindung ist die Vibrationsvorrichtung derart ausgebildet, dass damit gezielt Schwingungen mit variablen Frequenzen bzw. Frequenzbereichen erzeugt werden können. Bevorzugt wird bei dem Betreiben der Vibrationsvorrichtung mit einer hohen Frequenz gestartet, welche dann langsam abgesenkt wird. Die hohen Frequenzen zerstören kleinere Blasen, die dann koaleszieren und zu größeren Blasen werden. Größere Blasen werden durch tiefere Frequenzen angeregt. Auch die Erzeugung von Schwingungen in einem konstanten Frequenzbereich über einen bestimmten Zeitraum mit anschließender Änderung des Frequenzbereichs ist möglich.

Bevorzugt ist die Vibrationsvorrichtung zur Erzeugung von Hörschall ausgebildet.

Hörschall ist für Menschen hörbarer Schall mit einem Frequenzbereich von etwa 16 Hz bis etwa 16 kHz.

Für die Bekämpfung von Blasendispersionen kann eine Frequenz zwischen etwa 200 bis etwa 10.000 Hz, bevorzugt etwa 200 bis etwa 1.000 Hz gewählt werden. Für die Bekämpfung von Polyederschaum kann eine Frequenz im Bereich von etwa 25 bis etwa 1.000 Hz, bevorzugt 50 bis 500 Hz gewählt werden.

Nach einem zweiten Aspekt der vorliegenden Erfindung wird ein Verfahren zur Verringerung und/oder Verhinderung der Schaumproduktion und/oder zur Verbesserung der Entgasung vom Gärmedium in einem Biogasfermenter bereitgestellt, welches das Übertragen von Schwingungen auf den Fermenterinhalt umfasst.

Mit den Schwingungen des Hörschalls lässt sich auch Polyederschaum zerstören. Die Schwingungen (die Begriffe "Schwingungen" und "Vibrationen" werden hierin austauschbar verwendet) belasten die Lamellen des Schaums und führen so zu einem Platzen der Schaumblasen. Polyederschaum leitet die Schwingungsenergie nicht so gut wie eine Blasendispersion. Gegebenenfalls kann die Entschäumung an mehreren Stellen im Biogasfermenter durchgeführt werden.

Durch die Übertragung der Schwingungen auf den Fermenterinhalt kann die Produktion von Polyederschaum und Blasendispersionen sowohl verringert als auch verhindert werden. Der an der Oberfläche des Fermenterinhaltes befindliche Polyederschaum wird durch die Schwingungen zerschlagen. Die innerhalb des Gärmediums befindliche Blasendispersion wird dadurch bekämpft, dass durch die Schwingungen lokal gelöstes $CO_2$ aus dem übersättigten Gärmedium ausgetrieben wird und die Gasblasen koaleszieren. Dadurch vergrößern sich die Blasen und steigen aufgrund des erhöhten Auftriebs an die Oberfläche. Durch die anschließend entstehende Dichtedifferenz wird das entschäumte Medium durch neues schäumendes Medium ersetzt. Dies erlaubt eine lokal begrenzte Durchführung des Verfahrens.

Die Schaumzerstörung sollte kontinuierlich oder quasikontinuierlich erfolgen. Im Falle einer Blasendispersion dehnt sich das Gärmaterial mit der Gasbildungsrate aus (ca. 1 bis 10 $m^3$Gas/($m^3$ Reaktor und Tag), wenn die Schaumzerstörung ausbleibt. Als Folge kommt es in den meisten Biogasanlagen 30 Minuten bis einige Stunden nach dem Ansteigen des Gas Hold-Ups zu einem Beschädigen der Decke durch aufquellendes Gärmaterial. Der Prozess der Schaumzerstörung kann also nicht lange gestoppt werden. Ein Vorteil der vorgeschlagenen Methode ist jedoch der geringe Energiebedarf. Der Energieeintrag beträgt nur wenige hundert Watt. Ein Rührwerk hat hingegen ca. 10 kW, so dass der kontinuierliche Betrieb nicht weiter ins Gewicht fällt.

Zum Betreiben der Vibrationsvorrichtung gibt es mehrere Möglichkeiten. Die Vibrationsvorrichtung kann zum Beispiel (automatisch) durch Rückkopplung mit entsprechenden Schaummessgeräten aktiviert werden. Eine weitere und bevorzugte Möglichkeit ist die Vibrationsvorrichtung im Dauerbetrieb zu betreiben. Allerdings kann es notwendig sein, abhängig von der Art des Antriebs, Pausen zum Abkühlen des Antriebs einzulegen. Ebenso ist die Steuerung der Vibrationsvorrichtung mittels einer Zeitsteuerung möglich. Diese kann die Vibrationsvorrichtung in (un)regelmäßigen Zeitabständen deaktivieren und wieder aktivieren um so einerseits eine erhöhte Schaumproduktion und andererseits eine Überhitzung des Antriebs zu vermeiden. Hierbei können die Intervalle für die Pausen, in Abhängigkeit von dem Antrieb, von wenigen Sekunden bis hin zu mehreren Minuten lang sein. So sind zum Beispiel Pausen-Intervalle von 10, 20 oder 30 Sekunden bis zu 1, 2, 5, 10, 15, 20, 30 oder 60 Minuten denkbar.

Neben der Abhängigkeit des Betriebes der Vibrationsvorrichtung von dem Antrieb, hängt dieser auch sehr stark von dem Fermenterinhalt ab. So muss bei einem Fermenterinhalt mit starker Schaumproduktion der Betrieb entsprechend angepasst werden, indem, wenn überhaupt, weniger oder kürzere Pause eingelegt werden. Im Gegensatz dazu kann bei einem Fermenterinhalt mit geringer Schaumproduktion die Inbetriebnahme der Vibrationsvorrichtung verkürzt werden.

Gemäß der vorliegenden Erfindung wird ein Biogasfermenter zur Erzeugung von Biogas, in dem ein Gärmedium bis zu einem vorbestimmten Füllstand aufgenommen werden kann, dadurch gekennzeichnet, dass eine Vibrationsvorrichtung im Fermenter vorgesehen ist, bereitgestellt.

Durch die Verwendung einer entsprechenden Vibrationsvorrichtung wird durch die Übertragung der Schwingungen eine unkontrollierte Schaumbildung innerhalb des Biogasfermenters verhindert bzw. verringert. Durch das Anlegen bestimmter Frequenzen oder Frequenzbereiche können sowohl Polyederschäume als auch Blasendispersionen bekämpft werden. Dies verhindert, dass z. B. die Gasleitungen verstopft werden oder das Fermenterdach oder der montierte Gasspeicher beschädigt werden.

Solch ein Biogasfermenter ist mit zumindest einer Vibrationsvorrichtung oder auch mit mehreren gleichartigen oder verschiedenen Vibrationsvorrichtungen ausgestattet sein. Auch können die mehreren Vibrationsvorrichtungen gleiche oder unterschiedliche Frequenzen bzw. Frequenzbereiche erzeugen und auf das Gärmedium übertragen. Dadurch kann in einem Biogasfermenter parallel Schaumbildung unterschiedlicher Art bekämpft werden.

Die relative Position der Vibrationsvorrichtung bezüglich der Oberfläche des Gärmediums kann mit dem Füllstand variieren. Sie wird jedoch, wie auch der Füllstand, vorzugsweise konstant gehalten.

Weiterhin ist zu beachten, dass die Schaumschicht für die Vibrationsvorrichtung sehr korrosiv ist.

Die Vibrationsvorrichtung ist gemäß Anspruch 1 so im Fermenter angeordnet, dass sie sich immer im Gärmedium befindet. Wird ein horizontal angeordneter Schwingungsbalken als Vibrationsvorrichtung verwendet, dann ist es zweckmäßig, dass dieser immer vollständig im Gärmedium angeordnet ist.

Die auf das Gärmedium mittels der Vibrationsvorrichtung übertragenen Schwingungen bewirken zwei Effekte: i) Schaumzerstörung und ii) Entgasung. Die Schwingungen können Schaum zerstören, entweder durch Anregen der Eigenfrequenzen im Falle von Polyederschaum oder durch Lösen der Blasendispersionen im Gärmedium. In dem Bereich des Gärmediums, in dem die Vibrationsvorrichtung schwingt, wird das Gärmedium verflüssigt, d. h. dessen Viskosität wird verringert. Gleichzeitig fallen in den Bereich geringer Viskosität Feststoffe, wie Sand, (Fein-)Kies, Steine und Grit, aus und setzen sich nach unten ab.

Je weiter unten die Vibrationsvorrichtung im Fermenter angeordnet ist, also je größer der Abstand zur Oberfläche des Gärmediums ist, desto effizienter ist die Entgasung, denn gelöstes Gas bildet kleine Gasblasen, die aufsteigen und weiteres Gas aus den oberen Schichten des Gärmediums mitnehmen.

Um eine effiziente Entgasung des Gärmediums zu erzielen ist es zweckmäßiger die Vibrationsvorrichtung näher am Boden des Fermenters anzuordnen, wobei eine Anordnung in unmittelbarer Nähe des Bodens des Fermenters dazu führt, dass Feststoffe am Boden sedimentieren. Üblicherweise wird das Gärmedium im Fermenter mittels eines Rührwerkes umgewälzt. Durch das Umwälzen des Gärmediums werden Feststoffe am Boden aufgewirbelt und im Gärmedium dispergiert. Andererseits fallen grundsätzlich immer Feststoffe aus dem Gärmedium aus. Üblicherweise ist das Gleichgewicht zwischen Dispergieren und Ausfällen von Feststoffen in Biogasreaktoren so, dass sich am Boden keine Sedimentschicht von Feststoffen bildet. Ist die Vibrationsvorrichtung zu nah am Boden, dann fallen mehr Feststoffe aus, so dass mehr Feststoffe ausfallen als dispergiert werden können, wodurch sich am Boden eine Sedimentschicht von Feststoffen bildet. Dies ist sehr nachteilig, da dies zur Folge hätte, dass der Biogasfermenter in regelmäßigen Abständen am Boden gereinigt werden müsste, wozu er zu entleeren ist. Das Entleeren eines Biogasfermenters verursacht einen erheblich Betriebsausfall und Aufwand. Die Kosten hierfür sind beträchtlich.

Ordnet man die Vibrationsvorrichtung etwas höher an, dann fallen zwar auch im Bereich der Vibrationsvorrichtung die Feststoffe aus und senken sich in einem unteren Bereich des Gärmediums ab, in dem wieder eine höhere Viskosität vorliegt. Ein Großteil der Feststoffe wird dann wieder dispergiert. Dies gilt insbesondere, wenn das Gärmedium mittels eines Rührwerkes umgewälzt wird und in Bewegung ist. Hierdurch gelangen keine zusätzlichen oder nur geringe Mengen an zusätzlichen Feststoffen zum Boden des Fermenters und sedimentieren dort.

Erfindungsgemäß wird die Vibrationsvorrichtung mit einem Abstand von zumindest 0,5 m, insbesondere zumindest 1 m und vorzugsweise zumindest 1,5 m zum Boden des angeordnet, um ein Sedimentieren der Feststoffe am Boden des Fermenters zu verhindern und/oder sie sich gemäß Anspruch 1 im Bereich des mittleren Drittels oder des oberen Drittels der Höhe des Füllstandes im Biogasfermenter befindet.

Durch die Position der Vibrationsvorrichtung im Fermenter kann somit eingestellt werden, ob der schaumzerstörende Effekt oder der Entgasungseffekt stärker in Erscheinung tritt. Neben der Position der Vibrationsvorrichtung ist dies auch von der Art des Gärmediums abhängig (siehe unten).

Die erfindungsgemäße Vibrationsvorrichtung ist vor allem zum Zerstören von Schaum entwickelt worden. Sie kann jedoch unabhängig davon auch zum Entgasen des Gärmediums verwendet werden.

Für eine optimale Zerstörung des Schaumes insbesondere Polyederschaum, ohne zusätzlich nachteilige Effekte sollte die Vibrationsvorrichtung gemäß der vorliegenden Erfindung im Bereich 1/3 oder im zweiten Drittel der Höhe des Füllstandes angeordnet sein. Ein Anbringen von z. B. 0,5 m, 1 m oder 1,5 m unterhalb des Füllstandes erscheint zweckmäßig.

Gemäß der vorliegenden Erfindung ist bei einer Anordnung der Vibrationsvorrichtung im obersten Drittel der Höhe des Füllstandes der Effekt der Schaumzerstörung dominierend, wohingegen im mittleren Drittel der Effekt der Entgasung stärker ist. Das untere Drittel wird wegen der Gefahr der Sedimentbildung eher gemieden.

Für die genaue Betrachtung der Effekte muss vor allem zwischen den Medien unterschieden werden, in welchen möglicherweise entweder nur die Produktion von Polyederschaum oder nur von Blasendispersion zu erwarten ist. Im Falle eines Mediums, welches im Wesentlichen kein Polyederschaum produziert ist die Position der Vibrationsvorrichtung ausschlaggebend für dessen Wirkung. Wird die Vibrationsvorrichtung weit unterhalb der Oberfläche des Gärmediums angebracht ist die Entgasung durch die aufsteigenden Gasblasen erhöht, da diese mehr Strecke zurücklegen und dabei mehr Gasblasen mitnehmen können. Aufgrund der Zusammensetzung des Mediums wird sich auch an der Oberfläche kein zusätzlicher Polyederschaum bilden. Der Effekt der Entgasung und der Schaumzerstörung ist in solch einem Fall jeweils vorteilhaft für den Fermenterbetrieb. Eine Anbringung weiter unten im Fermenter, jedoch nicht näher als 0,5 m zum Fermenterboden wäre folglich sinnvoll, da sowohl der Prozess des Schaumzerstörens als auch der des Entgasens durch die Positionierung für den Betrieb nicht nachteilig wäre.

Handelt es sich hingegen um ein Gärmedium, welches im Wesentlichen nur Polyederschaum produziert, ist eine erhöhte Entgasung durch eine Positionierung weit unterhalb der Oberfläche des Gärmediums kontraproduktiv. Die verstärkt aufsteigenden Gasblasen würden zu einer erhöhten Menge an Polyederschaum an der Oberfläche führen. Folglich sollte im Falle solcher Medien die Vibrationsvorrichtung im oberen Drittel, im Wesentlichen zur Schaumzerstörung verwendet werden.

Ein direktes Schlagen des Schwingbalkens unterhalb der Oberfläche des Gärmediums des Fermenterinhaltes bewirkt eine verstärkte Bekämpfung von Polyederschaum. Die Übertragung der Schwingungen innerhalb des Gärmediums hingegen bewirkt in Abhängigkeit von der Reichweite der Schwingungen im Wesentlichen die Bekämpfung von Blasendispersionen.

Die oben beschriebenen unterschiedlichen Aspekte können auch in Kombination angewandt werden.

Die Erfindung wird nachfolgend anhand der beigefügten Zeichnungen beispielhaft näher erläutert. Die Zeichnungen zeigen in:

Fig. 1: Schematische Darstellung eines Biogasfermenters mit einem Schwingbalken als Vibrationsvorrichtungen zur Schaumbekämpfung;

Fig. 2: Schematische Darstellung eines Biogasfermenters mit einer Rüttelflasche als Vibrationsvorrichtungen zur Schaumbekämpfung;

Fig. 3: Schematische Darstellung einer erfindungsgemäßen Vibrationsvorrichtung (Schwingbalken);

Fig. 4: Schematische Darstellung einer erfindungsgemäßen Vibrationsvorrichtung (Rüttelflasche);

Fig. 5a: Schematische Darstellung (Draufsicht) einer erfindungsgemäßen Vibrationsvorrichtung (1-Modus Schwingbalken);

Fig. 5b: Schematische Darstellung (Seitenansicht) einer erfindungsgemäßen Vibrationsvorrichtung (1-Modus Schwingbalken);

Fig. 6: Resonanzfrequenz einer Biogasblasein einer typischen Fermenterflüssigkeit (Blasendispersion) als Funktion der Blasengröße ;

Fig. 7: Resonanzfrequenz einer Gasblase umgeben von einer Flüssigkeitslamelle (Polyederschaum) als Funktion der Blasengröße und des Schwingungsmodus (mehrere Modi abgebildet).

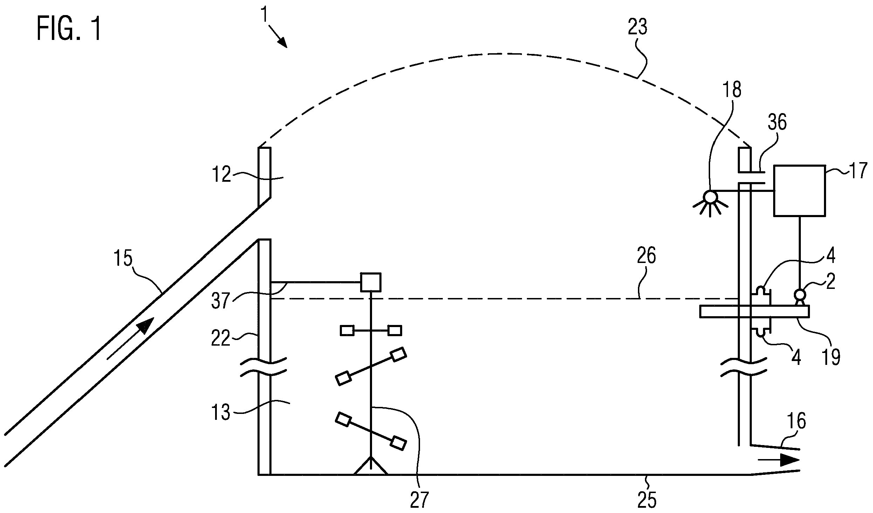

Eine erfindungsgemäße Vorrichtung 19 zur Verringerung der Schaumbildung ist in einem Biogasfermenter 1 vorgesehen (Fig. 1, 3). Der Biogasfermenter weist einen Fermenterboden 25, eine umlaufende Seitenwandung 22 und ein Foliendach 23 auf. Im vorliegenden Ausführungsbeispiel ist der Fermenter 1 zylinderförmig mit einer in Draufsicht kreisförmigen Seitenwandung 22 ausgebildet. Der Biogasfermenter weist ein Rührwerk 27 auf, mit welchem ein im Fermenter 1 enthaltenes Gärmedium 13, kontinuierlich umgewälzt werden kann. Derartige Rührwerke sind beispielsweise aus der DE 10 2005 041 798 A1, EP 14185121 A2, DE 20 2013 104 292 U1 und EP 14 191 283 A2 bekannt. Im folgenden Ausführungsbeispiel ist das Rührwerk 27 mittels eines Gestells 37 am Fermenter 1 befestigt.

Der Fermenter 1 ist mit einem Reaktorzulauf 15 versehen, mit dem zu vergärendes Gärmedium dem Fermenter 1 zugeführt wird. Der Reaktorzulauf 15 mündet am oberen Randbereich der Seitenwandung 22 in den Fermenter 1. Ein Reaktorablauf 16 ist im unteren Randbereich benachbart zum Fermenterboden 25 an der Seitenwandung 22 ausgebildet. Mit dem Reaktorablauf 16 kann vergorenes Gärmedium abgezogen werden.

Am oberen Randbereich der Fermenterwandung ist eine Gasableitung 36 angeordnet, mit welcher im Biogasfermenter 1 erzeugtes Biogas aus dem Biogasfermenter 1 abgeführt und beispielsweise einem Blockheizkraftwerk oder einer Einspeiseeinrichtung zum Einspeisen des Biogases in ein Gasnetz zugeführt wird.

Die Vorrichtung zur Verringerung der Schaumbildung ist in diesem Biogasfermenter als Vibrationsvorrichtung als Schwingbalkeneinrichtung 19 oder als Innenrüttler ausgebildet. Die Schwingbalkeneinrichtung 19 weist einen Schwingbalken 3 auf, der im vorliegenden Ausführungsbeispiel als Stahlbalken ausgebildet ist. Der Stahlbalken besitzt eine Länge von etwa 0,5 m bis etwa 1,5 m, eine Breite von etwa 5 cm bis etwa 20 cm und eine Dicke von etwa 1 cm. Der Schwingbalken 3 erstreckt sich horizontal durch eine Durchgangsöffnung 28 in der Seitenwandung 22 des Fermenters 1. Die Schwingbalkeneinrichtung 19 weist eine Buchse 6 auf, welche den Schwingbalken 3 umschließt und formschlüssig in die Durchgangsöffnung 28 der Seitenwandung 22 eingesetzt ist. Die Buchse 6 ist mittels eines Dichtelements 29 gegenüber der Seitenwandung 22 des Fermenters 1 abgedichtet.

Der Schwingbalken 3 wird mittels eines Federbalgs 4 in seiner Position bezüglich der Buchse 6 gehalten. Der Federbalg 4 ist aus einer elastischen Federbalgwandung 30 und einem Flanschblatt 7 ausgebildet. Die Federbalgwandung 30 ist aus einem Gummimaterial ausgebildet, das vorzugsweise faserverstärkt und/oder mit zumindest einem Drahtgewebe verstärkt ist, so dass die Federbalgwandung 30 einerseits eine gewisse Flexibilität besitzt und andererseits ausreichend steif ist, um den Schwingbalken 3 in einer etwa zentrischen Position zur Buchse 6 zu halten. Die Federbalgwandung 30 ist etwa rohrförmig ausgebildet weist im Querschnitt eine wellenförmige Kontur auf. Die Federbalgwandung ist mit einem rückwärtigen Endbereich mit dem Randbereich des Flanschblatts 7 mit einem umlaufenden Flanschelement 31 dicht verbunden. Der vorderseitige Randbereich der Federbalgwandung 30 ist mit zwei korrespondierenden Flanschelementen 32, 33 mit dem rückwärtigen Randabschnitt der Buchse 6 verbunden. Dieser rückwärtige Randabschnitt der Buchse 6 steht geringfügig an der Wandung 22 des Fermenters 1 nach außen vor.

Das Flanschblatt 7 weist eine Durchgangsöffnung auf, durch welche sich der Schwingbalken 3 erstreckt. Im zum Flanschblatt 7 benachbarten Bereich ist der Schwingbalken 3 mit einem Versteifungskörper 8 versehen, sowohl der Schwingbalken 3 als auch der Versteifungskörper 8 sind mit dem Flanschblatt 7 mechanisch fest und dicht verbunden. Die Verbindung kann als Schweißnaht oder auch als Vulkanisationsverbindung ausgebildet sein. Der Flansch 33 ist an ihrem inneren Randbereich mit der Buchse 6 über die Schweißnaht 40 verschweißt.

Am rückwärtigen Endbereich des Schwingbalkens 3 weist dieser einen Vibrationsantrieb 2 auf. Der Vibrationsantrieb ist ein an sich beliebiger Motor, der eine Exzenterscheibe antreibt, welche aufgrund ihrer Unwucht eine Vibration erzeugt. Da der Exzenterantrieb 2 fest mit dem Schwingbalken 3 verbunden ist, wird diese Vibration auf den Exzenterbalken 3 übertragen. Als Motor kann ein hydraulisch oder pneumatisch angetriebener Motor oder, da sich der Vibrationsantrieb 2 außerhalb des Fermenters 1 befindet, auch ein Elektromotor vorgesehen sein.

Der Schwingbalken 3 erstreckt sich nach innen in den Fermenter 1 und steht ein Stück an der Seitenwandung 22 nach innen vor.

Die Durchgangsöffnung 28 an der Innenwandung 22 des Fermenters 1 ist etwas unterhalb eines Füllstandes 26 des Fermenters 1 angeordnet. Im Betrieb wird der Fermenter 1 mit Gärmedium bis zum Füllstand 26 beladen, wobei der Füllstand einer gewissen Toleranz unterliegen kann. Die Durchgangsöffnung 28 und damit die Schwingbalkeneinrichtung 19 sind geringfügig unterhalb des minimalen Füllstandes angeordnet. Im Rahmen der Erfindung ist es auch möglich, die Schwingbalkeneinrichtung exakt auf Höhe des Füllstandes anzuordnen, wenn der Füllstand in Betrieb sehr genau geregelt wird. Bei einer solchen Ausführungsform kann der Schwingbalken mit seinem nach innen vorstehenden freien Ende 34 abwechselnd aus dem Gärmedium 13 herausbewegt werden und wieder in dieses eintauchen.

Wie bereits näher erläutert, hat die Anordnung der Schwingbalkeneinrichtung im Bereich benachbart zum Füllstand 26 des Gärmediums 13 den Vorteil, dass mit einer hohen Effizienz Schaum, insbesondere Polyederschaum, abgebaut werden kann. Außerdem ist es bei einer nachträglichen Anbringung im unteren Bereich des Fermenters wesentlich schwieriger die Vibrationsvorrichtung gegenüber dem vom Gärmedium ausgeübten Druck zuverlässig abzudichten.

An der Innenseite der Deckenwandung 21 oder über eine einfache Anbringung im oberen Bereich der Seitenwandungen 22 können ein oder mehrere Sensoren 18 angebracht werden, mit welchem die Höhe des Füllstandes 25 detektierbar ist und der Gas Hold-Up gemessen werden kann. Hierfür eignet sich insbesondere eine Kombination aus einem Ultraschall-Sensor (ermöglicht ein Messen durch die Schaumschichten) für die Füllstandmessung und einer Radarsonde zur Messung des Schaumes. Solche Sensoren sind, basierend auf ihren Funktionseigenschaften, so angeordnet, dass ihre Signale von oben nach unten in den Fermenter abgegeben werden.

Der Sensor 18 ist mit einer Steuereinrichtung 17 verbunden, die wiederum mit dem Vibrationsantrieb 2 der Schwingbalkeneinrichtung 19 verbunden ist. So kann über die Rückmeldung der Sensoren 18 zu der Steuereinheit 17 die Aktivierung und/oder die Frequenz der Vibrationsvorrichtung eigenständig reguliert werden.

Der oben beschriebene Fermenter wird mit dem jeweiligen Substrat als Gärmedium über den Reaktorzulauf befüllt und der Prozess der Biogaserzeugung wird gestartet. Wird über die angebrachten Sensoren 18 unkontrollierte Schaumbildung gemessen, wird das entsprechende Signal über die Steuereinrichtung an den Antrieb 2 der Vibrationsvorrichtung gesendet und diese aktiviert.

Wird ermittelt, dass sich der Füllstand nicht geändert hat, jedoch eine starke Schaumbildung gemessen wurde, so wird die Vibrationsvorrichtung dementsprechend mit einem Frequenzbereich zur Bekämpfung von Polyederschaum aktiviert. Wird hingegen eine Erhöhung des Füllstandes detektiert ohne, dass mehr Substrat in den Fermenter zugegeben wurde, so handelt es sich um Blasendispersionen. Dies hat zur Folge, dass die Vibrationsvorrichtung so aktiviert wird, dass Frequenzen erzeugt werden, die zur Bekämpfung der Blasendispersionen geeignet sind.

Im Falle, dass die Messung der Sensoren auf eine Produktion mehrerer Schaumarten schließen lässt, wird die Vibrationsvorrichtung mit sich ändernden Frequenzen aktiviert.

Das in den Fermenter ragende Ende 34 des Schwingbalkens 3 wird als Vibrationsübertragungselement bezeichnet. Dieses wird über lineare Schwingungen mittels dem Antrieb 2 in Bewegung versetzt und überträgt die Schwingungen auf den Fermenterinhalt, das Gärmedium 13.

Bei dem Schwingbalken 19 ist es zweckmäßig, dass der Schwerpunkt der Vibrationsvorrichtung in dem Flanschblatt 7 bzw. knapp dahinter liegt, so dass die Vibrationen nicht hauptsächlich den Federbalg 4 verformen, sondern vor allem auf das Gärmedium 13 übertragen werden.

Der angeregte Schwingbalken 3 überträgt dabei die Schwingungen auf das Gärmedium 13 indem er in das Medium eintaucht und er selbst im schwingenden Zustand das Medium zu keinem Zeitpunkt verlässt. Der Schwingbalken 3 schwingt komplett im Gärmedium, auf welches die Schwingungen übertragen werden. Diese Schwingungen bekämpfen dann in Abhängigkeit vom angesetzten Frequenzbereich und seiner Positionierung relativ zur Flüssigkeitsoberfläche 26 verschiedene Schaumarten oder aber bei kontinuierlicher Änderung des Frequenzbereichs mehrere Schaumarten.

Abhängig davon, ob der Füllstand konstant gehalten wird oder nicht, kann sich die Positionierung und damit verbunden die Art wie und an welcher Stelle die Schwingungen übertragen werden ebenfalls verändern. So würde ein absinkender Füllstand dazu führen, dass ein zunächst komplett unter der Füllstandoberfläche schwingende Schwingbalken zu einem späteren Zeitpunkt auf die Flüssigkeitsoberfläche aufschlägt und dadurch direkt auf Polyederschaum schlagen würde. Bei ansteigendem Füllstand wäre es entsprechend andersherum.

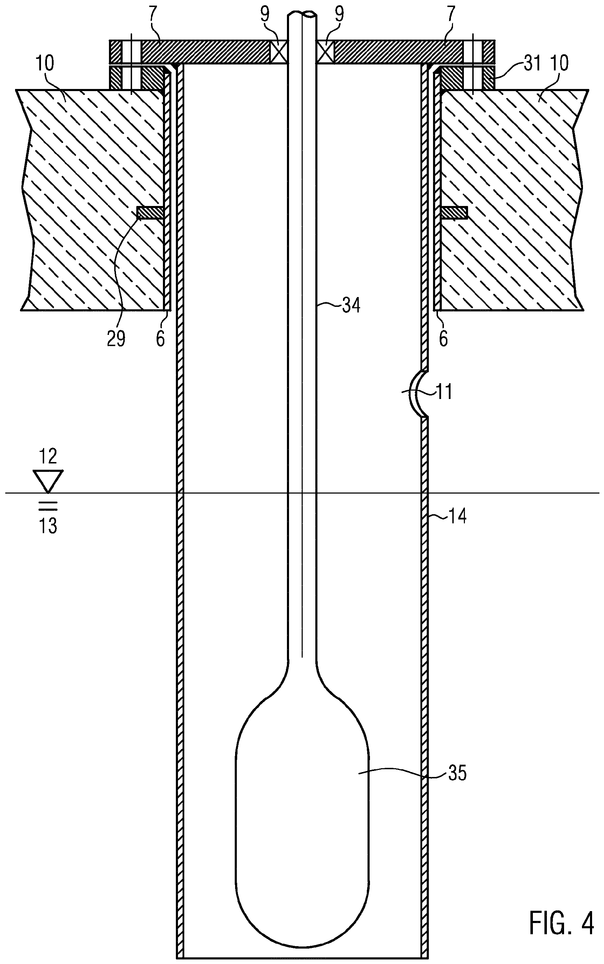

In einer weiteren Ausführungsform gemäß Anspruch 1 ist die Vorrichtung zur Verringerung der Schaumbildung in einem Biogasfermenter als Vibrationsvorrichtung und insbesondere als Innenrüttler (Rüttelflasche) 20 ausgebildet (Fig. 2, 4). Der Biogasfermenter weist einen ähnlichen Aufbau wie oben beschrieben auf (Fermenterboden 25, umlaufende Seitenwandung 22, Deckenwandung 21, Reaktorzulauf 15 und Reaktorablauf 16), weshalb hier auf eine genauere Beschreibung des Fermenters verzichtet werden kann. Die Deckenwandung 21 des Fermenters ist eine feste Fermenterdecke 10, in welcher der Innenrüttler angeordnet ist. Der Innenrüttler weist einen in den Fermenter ragenden Abschnitt auf, welcher als flaschenförmig angesehen werden kann. Dieser flaschenförmige Bereich umfasst einen oberen länglichen Teil 34, eine Art Flaschenhals, und einen unteren kugelähnlichen Bereich 35, den Flaschenkörper. Die Länge des oberen Bereiches 34 ist so gewählt, dass der untere Bereich 35 zumindest teilweise in das Gärmedium eintaucht. Abhängig von der Fermenterkonstruktion und der Befüllung des Fermenters kann die Länge des Innenrütlers entsprechend gewählt werden.

Der obere dünne Bereich 34 erstreckt sich vertikal durch eine Durchgangsöffnung 28 in der Fermenterdecke 10 des Fermenters 1. Die Innenrüttlereinrichtung 20 weist eine Buchse 6 auf, welche den Innenrüttler umschließt und formschlüssig in die Durchgangsöffnung 28 der Fermenterdecke 10 eingesetzt ist. Die Buchse 6 ist mittels eines Dichtelements 29 gegenüber der Fermenterdecke 10 des Fermenters 1 abgedichtet.

Am oberen Ende ragt der obere dünne Bereich 34 aus dem Fermenterinnenraum 5 hinaus. Das hinausragende Ende ist dort über ein entsprechendes Metallgerüst befestigt, damit der Innenrüttler zuverlässig gehalten wird. Die Durchgangsöffnung ist mittels einem Flanschblatt 7 abgedeckt, welches über ein umlaufendes Flanschelement 31 mit der Fermenterdecke 10 dicht verbunden ist. Das Flanschblatt 7 weist eine Öffnung auf, durch welche der obere Teil 34 des Innenrüttlers sich erstreckt. An der Schnittstelle des Innenrüttlers und dem Flanschblatt 7 ist eine Dichtung 9 vorgesehen, welche den Gasraum 12 gegenüber dem Fermenteraußenraum abdichtet, damit kein Gas aus dem Innenraum entweichen kann.

Der Innenrüttler besteht aus einer Unwucht, die über einen außenliegenden Elektromotor und einer flexiblen Welle im Kabel in Rotation versetzt wird. Es gibt auch pneumatische Innenrüttler, die mit Druckluft eine Unwucht in eine Kreisbewegung versetzen. Der pneumatische Rüttler hat Vorteile beim Ex-Schutz und der Kühlung. Hierbei läuft innerhalb des hohlen Innenrüttlers ein Druckluftschlauch (nicht dargestellt) um den Antrieb (nicht dargestellt) pneumatisch anzutreiben. Der Druckluftschlauch ist mit einem Kompressor verbunden, welcher sich außerhalb des Fermenters befindet.

Besonders bevorzugt sind Rüttler, die nur eine Linearbewegung erzeugen und somit die Lagerkräfte reduzieren. Solche Rüttler sind eine Kombination aus zwei gegenläufigen Unwuchtmotoren oder ein Piezoschwinger.

Der Teil des Innenrüttlers innerhalb des Fermenters befindet sich in einem nach unten offenen Führungsrohr 14, welches verhindert, dass klumpenartige im Gärmedium schwimmende Bestandteile mit dem Flaschenkörper 35 des Innenrüttlers in Kontakt kommen. Das Führungsrohr weist eine Entgasungsöffnung 11 auf um das entstandene Gas aus dem Führungsrohr in den Gasraum des Fermenters ausströmen lassen zu können.

Bevorzugt weist der Innenrüttler einen Spülanschluss auf, um bei einem Verstopfen des Rohres den Rüttler wieder zu befreien.

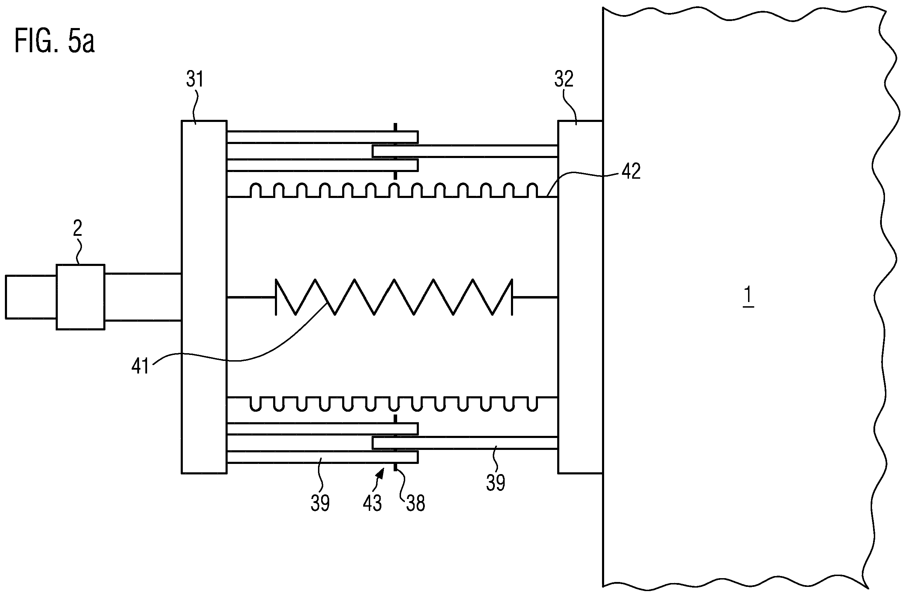

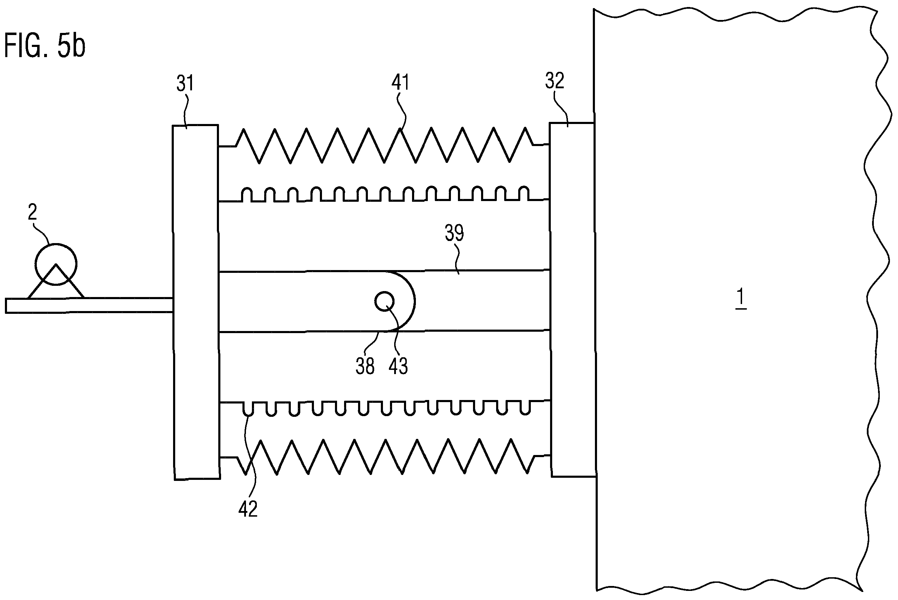

Eine weitere und bevorzugte Ausführungsform einer Vibrationsvorrichtung gemäß der vorliegenden Erfindung betrifft einen Schwingbalken wie oben beschrieben, welcher allerdings über eine ortsfeste Gelenk 43 mit einer Gelenkachse 38 gelagert und somit in seiner Beweglichkeit eingeschränkt wird. Bei einer horizontalen Gelenkachse kann der Schwingbalken nur in der vertikalen Ebene, sprich nur in einem Modus, schwingen. Solch eine Vibrationsvorrichtung wird hierin als "1-Modus Schwingbalken" bezeichnet und ist in Figuren 5a und 5b abgebildet.

Bevorzugt handelt es sich bei dem Antrieb eines 1-Modus Schwingbalkens um einen Piezoschwinger.

Der 1-Modus Schwingbalken ist gleichermaßen wie der oben beschriebene Schwingbalken in der Seitenwandung des Fermenters 1 angeordnet. Zusätzlich zu den Vorrichtungen des oben beschriebenen Schwingbalkens ist er über ein ortsfestes Gelenk 43 mit einer Gelenkachse 38 gelagert. Diese Lagerung wird mittels (Abstands-)Halterungen 39 außerhalb des Fermenters befestigt. Durch die Lagerung um die ortsfeste horizontale Gelenkachse 38 schwingt der Schwingbalken 3 nur in der vertikalen Ebene. Lediglich eine Aufwärts- und Abwärtsbewegung (Wipp-Bewegung) des Schwingbalkens 3 wird durch diese Anordnung zugelassen. Jegliche Bewegung entlang der horizontalen Ebene ist für diesen 1-Modus Schwingbalken ausgeschlossen. Grundsätzlich ist es jedoch auch möglich das ortsfeste Gelenk 43 nicht horizontal anzuordnen.

Die mittels dem 1-Modus Schwingbalken erzeugten Schwingungen sind somit leichter kontrollier- und steuerbar als die eines Schwingbalkens ohne ortsfeste Lagerung.

Die von dem 1-Modus Schwingbalken durchgeführten Schwingungen werden durch den Federbalg 4 (nicht dargestellt) einerseits abgefangen und andererseits zugleich auch unterstützt. Je nach Bewegung des Schwingbalkens 3 wird die eine Seite des Federbalges 4 zusammen gedrückt und erzeugt so eine Druckkraft, während die andere Seite des Federbalges 4 auseinander gezogen wird und dabei eine Zugkraft erzeugt. Anstelle eines Federbalges kann auch eine Schlauchverbindung (z.B. Wellschlauch, Metallschlauch, Gummischlauch, etc.)verwendet werden (Fig. 5a und 5b). Um in solch einem Fall dieselbe Zug- beziehungsweise Druckkraft zu erzeugen, können separate Zugfedern 41 angebracht werden, welche eine ähnliche Wirkung erzielen. Diese Federn werden bevorzugt außerhalb des Metallschlauchs angebracht.

Der Biogasfermenter 1 weist auch einen oder mehrere Sensoren 18, wie oben bereits beschrieben, auf, um den Füllstand und die Schaumproduktion zu messen. An der festen Fermenterdecke 10 sind die Sensoren 18 angebracht. Der Sensor ist mit einer Steuereinrichtung 17 verbunden, über welche die Vibrationsvorrichtung entsprechend den empfangenen Signalen angesteuert und aktiviert, deaktiviert oder ihre Frequenz geändert werden kann.

Nach dem Befüllen des Fermenters, wie bereits oben beschrieben, kann die Vibrationsvorrichtung verwendet werden um eine unkontrollierte Schaumproduktion zu bekämpfen. Hierfür ist der Innenrüttler entweder auf die gewünschte Höhe im Gärmedium einstellbar oder der Füllstand des Fermenters wird an die Höhe des Innenrüttlers entsprechend angepasst. Je nach Bedarf kann der Innenrüttler mit dem unteren dicken Bereich 35 in das Gärmedium unterschiedlich tief eingetaucht werden. Ergeben die Messungen durch die Sensoren 18, dass vor allem Blasendispersionen auftreten, so ist eine Schwingungsübertragung durch den Innenrüttler tiefer im Gärmedium zweckmäßig. Soll jedoch Polyederschaum an der Oberfläche bekämpft werden, so ist eine Übertragung der Schwingungen an der Oberfläche sinnvoll. Auch über das anregen mit verschiedenen Frequenzen oder Frequenzbereichen, wie oben beschrieben, können die verschiedenen Schaumarten bekämpft werden.

Bei einer vorteilhaften Ausführungsform wird ein Schwingbalken verwendet, der keine angulare Schwingung, sondern eine axiale Schwingung ausführt.

Dieser Schwingbalken besteht aus den Hauptkomponenten Universalkompensator, Vibrationsmotor, Gleitlager, Welle mit Scheibe und Abdichtungssystem.

Dieser Schwingbalken ist grundsätzlich so ausgebildet, dass am freien Ende des Schwingbalkens eine Scheibe angeordnet ist, welche durch die axiale Schwingung somit mit ihrer Breitseite auf die Flüssigkeit einwirkt.

Der Universalkompensator dient der Erzeugung der Rückstellung für das schwingungsfähige System in axialer Richtung. Des Weiteren ist in diesem Kompensator die Begrenzung der Schwingung in eine Richtung integriert.

Die Federkonstante des Metallbalges in axialer Richtung beträgt beispielsweise zwischen 100 und 150 N/mm$^2$, insbesondere 127 N/mm$^2$. Aus diesem Wert und der maximalen Amplitude von 2,5 mm, welche sich aus der Lebensdauerberechnung der Gleitlager ergibt, resultiert eine notwendige Vollfliehkraft von ca. 300 - 350 N für die Auslenkung des Kompensators. Alternativ zu einem Kompensator kann mit Federn gearbeitet werden, da es aufgrund der axialen Bewegung nicht notwendig ist, eine Abdichtung über den Kompensator zu realisieren.

Bei einer Amplitude in Luft von ca. 3,75 mm ergibt sich für den Vibrationsmotor eine Fliehkraft von etwa 475 N, um das System der gewünschten Amplitude in Bewegung zu setzen. Selbstverständlich sind auch größere Fliehkräfte möglich.

Bei dieser Ausführungsform ist es erforderlich, die Maschine mithilfe von zwei Gleitlagern zu lagern, so dass eine axiale Bewegung durchgeführt werden kann und alle sonstigen Massenkräfte aufgenommen werden können. Insgesamt ergibt sich auch eine erforderliche Mindestgröße für den Außendurchmesser der Welle.

Die Welle mit Scheibe ist so ausgelegt, dass sie eine wirkende dynamische Kraft von 100 bis 150 N in axialer Richtung ertragen kann. Bei dieser Variante wird die Schwingung nicht seitlich, d.h. angular ausgeführt, sondern in einer axialen Bewegung. Der entscheidende Vorteil ist eine gleichmäßige Verteilung der Schwinggeschwindigkeit über die gesamte Fläche der schwingenden Fläche, deren Geometrie frei gewählt werden kann. Hierdurch erfolgt eine gleichmäßige Kraftverteilung und eine günstige Auslegung des kompletten schwingenden Systems.

Dieser Schwingbalken ist vereinfacht so ausgebildet, dass eine mit zumindest einem Gleitlager gelagerte Welle an ihrem antriebsseitigen Ende mit einer Schwingeinrichtung verbunden ist. Die Schwingeinrichtung ist im vorliegenden Fall eine Aufnahme für mindestens einen Vibrationsmotor, vorzugsweise zwei Vibrationsmotoren, welche auf die Halterung wirken. Die Halterung und die Vibrationsmotoren sind hierbei beweglich federnd mit entsprechenden Federn an einer Fermenterwandung gelagert. Die Welle durchgreift die Fermenterwandung und besitzt an ihrem wasserseitigen freien Ende eine Scheibe, die entsprechend der axialen Schwingrichtung mit einer masseseitigen Breitseite und einer rückseitigen Fläche auf die Masse, die sie in Schwingung versetzt, entsprechend der Bewegungsrichtung einwirkt.

Insbesondere ist es zweckmäßig hierin beschriebene die Vibrationsvorrichtung nahe der Gasableitungen und Überdrucksicherungen zu installieren, um dadurch ein Verstopfen oder Beschädigen dieser zu vermeiden.

Die Zerstörung einer Blasendispersion mittels Hörschall ist am effizientesten, wenn die Frequenz der von außen eingetragenen Schwingung ungefähr der Resonanzfrequenz der Blasen entspricht. Die Resonanzfrequenz der Blasen lässt sich mit folgender Formel berechnen. Die Blasen schwingen dabei isotherm.

$$f_R = \frac{1}{D \cdot \pi} \sqrt{\frac{3}{\rho_L}\left(p + \frac{4 \cdot \sigma}{D}\right)}$$

Dabei ist

$f_R$   Resonanzfrequenz der schwingenden Blase
D   Blasendurchmesser
p   lokaler absoluter Druck (Hydrostatischer Druck plus Kopfdruck im Biogasfermenter)
$\sigma$   Oberflächenspannung
$\rho_L$   Dichte der Flüssigphase

Messungen der Oberflächenspannung in Gärmedien verschiedener Biogasanlagen ergaben geringfügig geringere Werte als in reinem Wasser gemessen wurde. Zur Messung der Oberflächenspannung wurde das Medium mit

einem 500 μm Spaltensieb gesiebt. Die Oberflächenspannung korreliert dabei mit der Viskosität des gesiebten Gärmediums. Je höher die Viskosität des gesiebten Gärmediums ist, desto höher ist die Oberflächenspannung (siehe Tabelle 1 für verschiedene Substrate mit unterschiedliche Verweilzeiten). Die Viskosität des ungesiebten Gärmediums ist um ein vielfaches höher und liegt bei Werten zwischen 5 und 100 Pas (bei einer Scherrate von 10 s$^{-1}$).

Tabelle 1 zeigt die Viskosität von verschiedenen Substratzusammensetzungen je nach Verweilzeit im Fermenter und der damit zusammenhängenden Oberflächenspannung.

| Anlage | Substrate | Verweilzeit Fermenter [d] | Viskosität gesiebtes Gärmedium (500 μm) bei 20 °C und 20 s$^{-1}$ [mPas] | Oberflächenspannung bei 17 °C in mN/m |
|---|---|---|---|---|
| 1 | Maissilage | 35 | 246 | 76,2 |
| 2 | Maissilage | 45 | 196 | 74,6 |
| 3 | Maissilage | 28 | 227 | 74,6 |
| 4 | Maissilage | 30 | 474 | 76,7 |
| 5 | Maissilage | 13 | 41 | 71,3 |
| 6 | 90% Maisilage, 10% Grassilage | 15,5 | 405 | 81,9 |
| 7 | 75% Maissilage, 25% Hähnchenmist | 14 | 646 | 81,7 |
| 8 | 25% Maissilage, 75% Grassilage | 60 | 18 | 72,4 |
| Wasser | | | 1 | 81,9 |

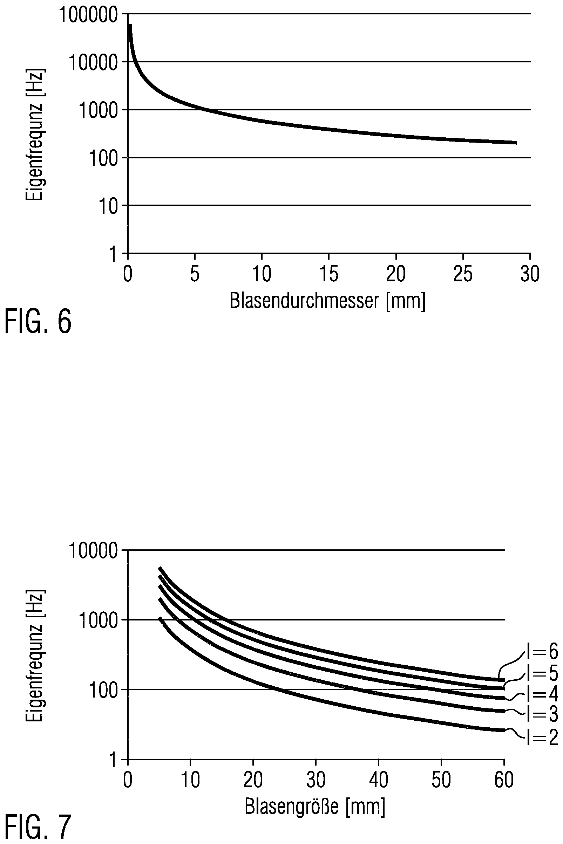

Figur 6 zeigt die Eigen- bzw. Resonanzfrequenz einer Biogasblase in einer typischen Fermenterflüssigkeit (Blasendispersion) als Funktion der Blasengröße. Die typische Blasengröße im Biogasprozess liegt bei 1 mm bis 10 mm. Koaleszierte Blasen können bis zu 100 mm groß werden. Zur Bekämpfung von Blasendispersionen ist daher der Bereich 600 Hz bis 10000 Hz zu bevorzugen.

Anders als bei einer Blasendispersion ist die Gasphase im Polyederschaum nicht von Flüssigkeit sondern ebenfalls von Gas umschlossen. Das Resonanzverhalten von Polyederschaum kann in erster Näherung mit dem Resonanzverhalten von Seifenblasen beschrieben werden. Die geringste Eigenfrequenz bei einer definierten Blasengröße ist die Grundschwingung, bei der auch die größte Amplitude auftritt. Andere Modi erzeugen geringere Amplituden. Typischer Polyederschaum hat eine Blasengröße von 10 mm bis 50 mm. Zur Bekämpfung von Polyederschaum ist daher eine Frequenz von 30 bis 300 Hz zu bevorzugen.

In Figur 7 wird die Eigen- bzw. Resonanzfrequenz einer Gasblase umgeben von einer Flüssigkeitslamelle (Polyederschaum) als Funktion der Blasengröße und des Schwingungsmodus dargestellt (mehrere Modi abgebildet).

Die Eigenfrequenzen einer Seifenblase lauten (Kornek et al, Oscillations of soap bubbles, New Journal of Physics 12 (1010) 073031 pp21):

$$f_R = \frac{1}{2 \cdot \pi} \sqrt{\frac{16 \cdot \sigma}{D^3 \cdot \rho_G} \frac{(l-1) \cdot l \cdot (l+1) \cdot (l+2)}{2l+1}}$$

Dabei ist

$\rho_G$    Dichte des Biogases in der Schaumschicht (ca. 1,1 kg/m$^3$)
$l$    Schwingungsmodus

Die Blasengröße bei Polyederschaum in Biogasanlagen ist typischerweise 10 bis 50 mm. Die Eigenfrequenzen von Schaum für $l$ = 2, 3, 4, 5 und 6 sind in Figur 7 dargestellt. Es zeigen sich minimale Eigenfrequenzen ($l$ = 2) von 50 bis 1000 Hz je nach Blasengröße. Höhere Schwingungsmodi (vergleichbar mit Obertönen bei einem beidseitig eingespannten Balken) erlauben höhere Resonanzfrequenzen. Für die Zerstörung von Polyederschaum in Biogasanlagen sind Frequenzen von etwa 25 bis etwa 1000 Hz zweckmäßig. Je größer die Blasen sind, umso geringer sollte die Frequenz der Vibrationsvorrichtung bzw. eines Vibrationsübertragungselementes sein.

Insbesondere eignet sich die Vibrationsvorrichtung der vorliegenden Erfindung für Biogasanlagen mit einem viskosen- oder hochviskosen Gärmedium. Solche Medien weisen einen Feststoffanteil von >10% auf. Solche hochviskosen Medien weisen hohe Widerstandskräfte auf. Kleine Gasblasen, die nur eine geringe Auftriebsgeschwindigkeit besitzen, erzeugen somit nur eine geringe Scherrate und sind von dieser hochviskosen Flüssigkeit umgeben.

Generell ist der Einsatz der Vibrationsvorrichtung in allen Biogasanlagen geeignet, in welchen schaumbildende Substrate eingesetzt werden sollen oder, in welchen sich ein hochviskoses Gärmedium ausbildet. Je nach Art des Gärmediums kann es zur Bildung von Polyederschaum oder Blasendispersionen neigen. Ein solches Medium stellt sich bei typischen Biogasanlagen ab einem Trockenrückstand von etwa 10% und mehr im Fermenter ein.

Des Weiteren eignet sich die Vibrationsvorrichtung der vorliegenden Erfindung insbesondere für Biogasanlagen, welche als Substrate, Zuckerrüben, Zuckerrübenpressschnitzel, Getreide, Getreideganzpflanzensilage, Abfälle wie Speisereste, Fettabscheider, Fette, Blut, Schlachtabfälle, Bioabfall und/oder Mist, insbesondere Pferdemist verwenden.

In einer bevorzugten Ausführungsform der vorliegenden Erfindung werden die Schwingungen nahe der Oberfläche des Fermenterinhaltes in das Gärmedium eingebracht. Bei einer Einbringung in tiefere Schichten können die Vibrationen die Sedimentation von Schweb-/Feststoffen verstärken. Schweb-/Feststoffe, wie zum Beispiel Sand, Feinkies oder Grit, sedimentieren dann nur im oberen Bereich des Behälters, lagern sich aber nicht am Boden ab.

Allerdings ist zu berücksichtigen, dass die genaue Positionierung der Vibrationsvorrichtung nicht nur von der Positionierung der Vorrichtung selber, sondern auch von dem Füllstand des Fermenters abhängt. Der Füllstand kann entweder konstant gehalten werden, was zur Folge hat, dass die Vorrichtung (kleine Schwankungen des Füllstandes sind nicht auszuschließen) hinsichtlich ihrer Positionierung relativ zur Oberfläche des Fermenterinhaltes ebenfalls konstant bleibt. Durch Steuerung des Füllstandes lässt sich somit auch die Positionierung der Vibrationsvorrichtung bezüglich einer Schaumschicht oder des ungeschäumten Gärmediums beeinflussen. Jedoch kann der Füllstand meist nicht beliebig variiert werden, sondern wird bei kontinuierlich betriebenen Reaktoren im Allgemeinen auf einen festen Wert geregelt.

Bei der Verwendung einer Vibrationsvorrichtung ist diese so angeordnet, dass sie entweder geringfügig oberhalb der Oberfläche des Gärmediums, aber noch innerhalb des Polyederschaumes, unterhalb oder direkt auf der Oberfläche liegt. Dies kann, wie oben beschrieben, abhängig vom Füllstand variieren oder aber konstant gehalten werden.

Die genaue Anordnung, insbesondere die genaue Positionierung bezüglich die Oberfläche des Fermenterinhaltes, der Vibrationsvorrichtung hängt von der Art der verwendeten Vorrichtung ab.

Durch die Schwingungen, welche von dem Schwingbalken ausgehen wird nicht nur der unmittelbar an die Vorrichtung angrenzende Bereich erreicht, sondern auch ein umliegender Bereich in der Nähe der Vibrationsvorrichtung. Die Reichweite bzw. Größe dieser Zone um den Schwingbalken hängt von mehreren Faktoren, wie Anregung, Frequenz, Durchmischung des Gärmediums, Art des Gärmediums etc. ab.

Somit bewirkt zum Beispiel auch eine unterhalb des Fermenterinhaltoberfläche positionierte Vibrationsvorrichtung, dass Polyederschaum oberhalb des Füllstandes durch die erzeugten weitreichenden Frequenzen zerstört wird.

Je nach Art der Vibrationsvorrichtung kann diese an einer der Fermenterwandungen (Seitenwände oder Decke) angebracht sein. Bei der Verwendung von Rüttelflaschen, welche senkrecht von der Decke in den Fermenterinhalt eingetaucht werden, ist eine Anbringung an der Fermenterdecke, sowie über ein (separat) montiertes Gestell möglich.

Eine bevorzugte Ausführungsform des Schaumzerstörers ist die Ausführung mit einem 1-Modus Schwingbalken. Dieser weist nur einen Freiheitsgrad auf und kann beispielsweise mit Federn vorgespannt werden oder als Wellrohrkompensator ausgeführt sein.

Um das Resonanzverhalten dieser Schwinger zu untersuchen muss die Eigenfrequenz der ungedämpfen Schwingung betrachtet werden. Für den 1-Modus Schwingbalken ist diese

$$\omega_0 = \sqrt{\frac{C \cdot r^2}{J}}$$

Mit

C    [N/m] Federkonstante
r    [m] Radius des Flanschblatts
J    [kg m$^2$] Trägheitsmoment des Schwingers

Für einen Schaumzerstörer, der aus einem 1 m langen Balken mit der Dicke 1 cm und der Breite von 10 cm und einem DN200 Flanschblatt besteht und aus Edelstahl gefertigt ist, beträgt das Trägheitsmoment ca. 3 kg m$^2$. Herkömmliche Zugfedern mit einem Durchmesser von 2 cm weisen eine Federkonstante von etwa 300 N/m auf. Die Eigenfrequenz der ungedämpften Schwingung beträgt somit ungefähr 1 s$^{-1}$.

Bei einer Fremdanregung mit 50 Hz oder mehr kann es daher nicht zu einer Amplitudenerhöhung oder gar einer Resonanzkatastrophe kommen.

Betrachtet man den Balken als starr, lautet die Bewegungsgleichung des eingeschwungenen Systems:

$$\alpha(t) = \frac{\gamma \cdot \sqrt{(\omega_0{}^2 - k^2)^2 + 4\delta^2 k^2}}{4\delta^2 k^2 + (k^2 - \omega_0{}^2)} \cdot sin(kt + \varphi)$$

Mit

$$\gamma = -\left(\frac{a}{2} + c\right) \cdot \frac{m_M \cdot r_M}{J} \cdot k^2$$

$$\varphi = \arctan\left(\frac{(\omega_0{}^2 - k^2)}{2 \cdot \delta \cdot k}\right)$$

$$\delta = \frac{D}{2J}$$

$$D = \frac{1}{4}b^3 \cdot \frac{\tau_0 \cdot K}{\dot{\gamma}_{max}} + \frac{1}{16}a^2 \cdot b \cdot \frac{\tau_0 \cdot K}{\dot{\gamma}_{max}}$$

| | |
|---|---|
| $\gamma$ | [Nm] Erregungsdrehmoment, Zentrifugalkraft des Unwuchtmo tors mal Hebelarm |
| $\varphi$ | [rad] Phasenwinkel zwischen Erregung und Schwingung |
| $\delta$ | [s-1] Kenngröße zur Unterscheidung zwischen Schwing- und Kriechfall |
| D | [Nms] Dämpfungskonstante |
| a | [m] Länge Kompensator |
| b | [m] Länge Schwingbalken |
| c | [m] Abstand Unwuchtmotor von Flanschplatte |
| k | [s^{-1}] Erregerfrequenz |
| $m_M \cdot r_M$ | [kg m] Arbeitsmoment des Unwuchtmotors |
| $\tau_0$ | [Pa] Fließgrenze des Schäuemdnen Mediums (Bingham-Fluid) |
| $\dot{\gamma}_{max}$ | [s^{-1}] maximale Scherrate am Ende des Schwingbalkens |
| K | [-] Korrelationskonstante zwsichen $c_w$-Wert und Re-1 (unge fähr 100 für einen langen Balken) |
| A | [rad] Amplitude (Term vor dem Sinus in der Bewegungsglei chung) |

Die Bewegungsgleichung gilt vor allem für kleine Winkel ( ⸱ ▪ $\leq$ 0,1). Die Leistung der Schwingung ist

$$P = \delta \cdot J \cdot k^2 \cdot A(k)$$

Soll der Schaumzerstörer bei höheren Frequenzen angeregt werden, steigt die benötigte Leistung mit der vierten Potenz an. Limitiert man die Leistung des Erregers ergibt sich die Amplitude unmittelbar aus der letztgenannten Gleichung.

Schwingt der Schaumzerstörer in der Luft, entfällt die Dämpfung. Die Amplitude kann dann sehr groß werden. Die Amplitude sollte daher überwacht werden und der Schaumzerstörer abgeschaltet werden, wenn die Amplitude über einen Grenzwert steigt.

Bei dem Antrieb 2 kann es sich um einen luftdruckgesteuerten pneumatischen Antrieb, einen Asynchronmotor oder um einen Unwuchtmotor handeln. Mithilfe von z.B. einem Frequenzumrichter können unterschiedliche Frequenzen erzeugt werden können.

Die Frequenzen können kontinuierlich in einem bestimmten Bereich geändert werden (Wobbeln) oder es kann in der Art eines Frequenzmultiplex zwischen mehreren einzelnen Frequenzen geschaltet werden, die jeweils für eine vorbestimmte Zeitdauer ausgeführt werden.

Ein Anbringen außerhalb des Fermenters ist ebenfalls vorteilhaft, wenn der Antrieb 2 ausgetauscht oder gewartet werden soll. Auch wird dadurch verhindert, dass der Antrieb 2 den Bedingungen innerhalb des Fermenters ausgesetzt ist, was wiederum seine Lebensdauer erhöhen kann.

Der Einsatz einer Vibrationsvorrichtung an der Fermenterwandung nahe der Oberfläche hat zur Folge, dass dieser oftmals in einer Ex-Zone lokalisiert sein wird, wenn ein Gasspeicher auf dem Fermenter angeordnet ist. Die technische Information Nr. 4 (TI4, Sicherheitsregeln für Biogasanlagen) der landwirtschaftlichen Berufsgenossenschaft in Deutschland beschreibt, dass sich eine Ex-Zone 2 bis 2 m unterhalb der Fermenterkrone ausdehnt. Andere Länder haben andere Anforderungen an die Explosionssicherheit. Eine Ausführung der Vibrationsvorrichtung zum Einsatz in einer Ex-Zone ist jedoch erstrebenswert. Dies kann durch den Einsatz von pneumatischen Rüttlern erreicht werden. Ein pneumatischer Rüttler ist beispielsweise ein Kugelrüttler oder Rollenrüttler, bei dem ein Druckluftstrom eine Kugel auf einer bestimmten Bahn bewegt und somit eine Vibration herbeiführt. Diese Rüttler laufen oft bei höheren Frequenzen von 100 Hz und mehr.

Vorzugsweise sind die Vibrationsvorrichtungen der vorliegenden Erfindung derart gestaltet, dass eine Nachrüstung in Bestandsbiogasanlagen ohne größeren Aufwand möglich ist. Die genaue Ausgestaltung der Vibrationsvorrichtung kann je nach Biogasfermenter dementsprechend angepasst sein. Insbesondere die Anbringung der Vibrationsvorrichtung erfordert möglicherweise Maßnahmen, welche von dem jeweiligen Biogasfermenter abhängen.

Die Art der Vibrationsvorrichtung, welche in einem Biogasfermenter verwendet wird, kann je nach Bedarf, in Abhängigkeit von dem Fermenterinhalt, entsprechend gewählt werden. Auch können ein oder mehrere Vibrationsvorrichtungen unterschiedlicher und/oder der gleichen Art an einem Biogasfermenter angebracht werden.

Bei Polyederschaum (z.B. in Gärmedien enthaltend Zuckerrüben, Zuckerrübenpressschnitzel, Schlachtabfälle, Blut, Roggen- oder Ganzpflanzensilage) ist eine hohe Resonanzfrequenz mit geringer Dämpfung, bei einer hohen Einbau-Position empfehlenswert.

Bei Blasendispersion (z.B. in Gärmedien enthaltend Mist oder Bioabfall) hingegen empfiehlt sich eine geringere Resonanzfrequenz mit hoher Dämpfung, bei eingetauchte Einbauposition.

Bei den oben erläuterten Ausführungsformen wird zum Detektieren des Füllstandes und der Höhe des Schaumes eine Kombination aus einem Ultraschallsensor und einer Radarsonde verwendet. Alternativ können hierzu auch andere Sensoren und Sonden verwendet werden, die zum Detektieren der Höhe des Füllstandes und/oder eines Schaumpegels geeignet sind. Solche Sensoren und Sonden sind beispielsweise Schwinggabeln, Leitfähigkeitssonden, Drehsonden, kapazitive Sonden oder Schwimmer-Schalter. Weiterhin können Sonden zur Durchführung von hydrostatischen Messungen verwendet werden. Wird der hydrostatische Druck in unterschiedlichen Höhen im Fermenter gemessen, so kann hieraus der Gasanteil (Gas Hold-Up) im Gärmedium abgeleitet werden. Die Dichte des Gärmediums ohne Gasanteil ist bekannt. Aus dem aktuell gemessenen Füllstand kann anhand des hydrostatischen Druckes bestimmt werden, welche mittlere Dichte das Gärmedium oberhalb des Messpunktes Druck aufweist. Durch Messungen des hydrostatischen Druckes in mehreren Höhen kann somit die tatsächliche mittlere Dichte berechnet werden, wodurch sich für die einzelnen Schichten zwischen den Messpunkten der Gasanteil bestimmen lässt.

Die Vibrationsvorrichtung kann an die Strom- und Notstromversorgung angeschlossen werden, wodurch die Biogasanlage eigensicher gegenüber Schaumereignissen wird, ohne dass der Schaumzerstörer in die Anlagensteuerung integriert werden muss. Die erfindungsgemäße Vibrationsvorrichtung eignet sich insbesondere für kleine landwirtschaftliche Anlagen, die oftmals nur einen geringen Automatisierungsgrad aufweisen.

Grundsätzlich werden beim erfindungsgemäßen Verfahren mittels einer Vorrichtung Hörschall auf den Fermenterinhalt übertragen, wodurch verschiedene Arten von Schaum (z. B. Polyederschaum und Blasendispersionen) verhindert oder aus dem Fermenterinhalt entfernt werden können.

Der an der Oberfläche des Fermenterinhaltes befindliche Polyederschaum wird durch die Schwingungen sowohl mechanisch als auch in naher Umgebung durch eine herbeigeführte Resonanzkatastrophe zerstört. Die innerhalb des Gärmediums befindliche Blasendispersion wird dadurch bekämpft, dass durch die Schwingungen lokal gelöstes $CO_2$ aus dem übersättigten Gärmedium ausgetrieben wird und die Gasblasen koaleszieren. Dadurch vergrößern sich die Blasen und steigen aufgrund des erhöhten Auftriebs an die Oberfläche. Durch die anschließend entstehende Dichtedifferenz wird das entschäumte Medium durch neues schäumendes Medium ersetzt. Dies erlaubt eine lokal begrenzte Durchführung des Verfahrens.

Für die Bekämpfung von Polyederschaum kann es jedoch möglich sein, dass eine lokal begrenzte Durchführung nicht ausreichend ist, da dieser die Schwingungsenergie nicht besonders gut leitet.

Für die Bekämpfung von Blasendispersionen wird das Verfahren bevorzugt mit einer Hörschallfrequenz von etwa 200 Hz bis etwa 10.000 Hz, bevorzugt etwa 200 Hz bis etwa 1.000 Hz durchgeführt.

Polyederschaum wird mit einer Frequenz von etwa 25 Hz bis etwa 10.000 Hz, bevorzugt etwa 50 Hz bis etwa 500 Hz bekämpft.

In einer bevorzugten Ausführungsform wird die Frequenz variiert. Dies kann sowohl bedeuten, dass mit einer hohen Frequenz gestartet wird und diese kontinuierlich abgesenkt wird oder, dass über längere Zeiträume unterschiedliche Frequenzen alternierend oder auch in Superposition konstant angelegt werden.

Bevorzugt wird das Verfahren mit einer Vibrationsvorrichtung, wie oben beschrieben, durchgeführt.

In einer bevorzugten Ausführungsform umfasst der Biogasfermenter auch eine Vorrichtung zur Messung der Schaumbildung innerhalb des Fermenters, welche an die Vibrationsvorrichtung gekoppelt ist und entweder direkt an dieser angebracht oder an einer separaten Stelle im Fermenter montiert ist. Dies hängt davon ab, welche Art von Messvorrichtung verwendet wird.

In einer bevorzugten Ausführungsform der vorliegenden Erfindung kann ein bereits existierender und möglicherweise in Betrieb genommener Biogasfermenter auf einfach Art und Weise mit der entsprechenden Vibrationsvorrichtung nachgerüstet werden.

Auch muss die Vibrationsvorrichtung zur Bekämpfung des Schaums nicht zwangsläufig in die Anlagensteuerung integriert werden. Vielmehr kann diese über die Vorortsteuerstelle angesteuert werden. Wird das Signal der oben beschriebenen Sonden zur Messung des Füllstandes und/oder der Schaumbildung ebenfalls auf der Vorortsteuerstelle aufgelegt, wird die Biogasanlage eigensicher gegenüber Schaumereignissen. Solch ein Biogasfermenter reguliert sich selbstständig gegenüber Schaumereignissen und kann durch Anschließen der Vibrationsvorrichtung(en) an die Strom- und Notstromversorgung unabhängig von der Anlagensteuerung installiert werden. Dadurch lassen sich Biogasanlagen einfach mit den hierin beschriebenen Vorrichtungen zur Schaumbekämpfung nachrüsten und erfüllen dann den geforderten Stand der Technik bezüglich Eigensicherheit im Sinne einer Genehmigung nach dem Bundes-Immissionsschutzgesetz.

Die vorliegende Erfindung erlaubt es, Biogasanlagen bereitzustellen, welche ohne übermäßigen Aufwand und kostengünstig mit den entsprechenden Vorrichtungen nachgerüstet werden können, um dadurch Biogasanlagen zu erhalten, welche die Schaumbildung innerhalb des Fermenters verhindern bzw. verringern und/oder die Entgasung des Gärmediums verbessern

Bezugszeichenliste

| | | | |
|---|---|---|---|
| 1. | Fermenter | 23. | Foliendach |
| 2. | Antrieb | 24. | Tauchrohr |
| 3. | Schwingbalken | 25. | Fermenterboden |
| 4. | Federbalg | 26. | Flüssigkeitsoberfläche |
| 5. | Fermenterinnenraum | 27. | Rührwerk |
| 6. | Buchse | 28. | Durchgangsöffnung |
| 7. | Flanschblatt | 29. | Dichtelement |
| 8. | Versteifungskörper | 30. | Federbalgwandung |
| 9. | Dichtung | 31. | Flanschelement |
| 10. | Fermenterdecke | 32. | Flanschelement |
| 11. | Entgasungsöffnung | 33. | Flanschelement |
| 12. | Gasraum | 34. | Oberer Bereich des Innenrüttlers |
| 13. | Gärmedium | 35. | Unterer Bereich des Innenrüttlers |
| 14. | Führungsrohr | 36. | Gasableitung |
| 15. | Reaktorzulauf | 37. | Gestell |
| 16. | Reaktorablauf | 38. | Gelenkachse |
| 17. | Steuereinrichtung | 39. | Halterung |
| 18. | Sensor | 40. | Schweißnaht. |
| 19. | Schwingbalkeneinrichtung | 41. | Zugfeder |
| 20. | Innenrüttler | 42. | Schlauchverbindung |
| 21. | Deckenwandung | 43. | Gelenk |
| 22. | Seitenwandung | | |

**Patentansprüche**

1. Fermenter zur Erzeugung von Biogas, in dem ein Gärmedium bis zu einem vorbestimmten Füllstand aufgenommen werden kann, wobei eine Vibrationsvorrichtung im Fermenter vorgesehen ist, wobei es sich bei der Vibrationsvorrichtung um einen Schwingbalken oder einen Innenrüttler handelt, **dadurch gekennzeichnet, dass** das Schwingungselement zu einem Boden des Fermenters einen Abstand von zumindest 0,5 m aufweist und/oder sich im Bereich des mittleren Drittels oder des oberen Drittels der Höhe des Füllstandes im Biogasfermenter befindet.

2. Fermenter nach Anspruch 1, **dadurch gekennzeichnet, dass** der Schwingbalken axiale Schwingungen oder angulare Schwingungen ausführend ausgebildet ist und/oder dass die Vibrationsvorrichtung zur Erzeugung variabler Frequenzen ausgebildet ist.

3. Fermenter nach Anspruch 1 oder 2, **dadurch gekennzeichnet, dass** die erzeugten Schwingungen Hörschall sind.

4. Fermenter nach einem der Ansprüche 1 bis 3, **dadurch gekennzeichnet, dass** die Vibrationsvorrichtung einen Antrieb und ein Vibrationsübertragungselement aufweist.

5. Fermenter nach Anspruch 4, **dadurch gekennzeichnet, dass** der Antrieb außerhalb des Biogasfermenters angeordnet ist und/oder dass der Antrieb ein pneumatischer Antrieb, ein Asynchronmotor, ein Unwuchtmotor oder ein Piezoschwinger ist.

6. Fermenter nach einem der vorhergehenden Ansprüche, **dadurch gekennzeichnet, dass** die erzeugte Frequenz zum Bekämpfen von Polyederschaum zumindest 16 Hz, vorzugsweise 25 Hz bis 1000 Hz und insbesondere zumindest 50 Hz bis 500 Hz beträgt.

7. Fermenter nach einem der Ansprüche 1 bis 6, **dadurch gekennzeichnet, dass** die erzeugte Frequenz zum Bekämpfen der Blasendispersion nicht mehr als 16 kHz, insbesondere nicht mehr als 10 kHz oder nicht mehr als 100 kHz und vorzugsweise nicht mehr als 500 Hz beträgt.

8. Ferenter nach einem der Ansprüche 1 bis 7, **dadurch gekennzeichnet, dass** es sich bei der Vibrationsvorrichtung um einen 1-Modus Schwingbalken handelt.

9. Verfahren zur Verringerung der Schaumproduktion und/oder Verbesserung der Entgasung in einem Biogasfermenter, wobei Schwingungen mit einem Schwingbalken oder Innenrüttler auf ein im Biogasfermenter befindliches Gärmedium übertragen werden, **dadurch gekennzeichnet, dass** das Schwingungselement zu einem Boden des Fermenters einen Abstand von zumindest 0,5 m aufweist, und/oder im Bereich des mittleren Drittels oder des oberen Drittels der Höhe des Füllstandes im Biogasfermenter angeordnet wird.

10. Verfahren nach Anspruch 9, **dadurch gekennzeichnet, dass** der Schwingbalken in axiale-Schwingungen oder angulare Schwingungen versetzt wird und/oder dass das Schwingungselement Schwingungen mit variabler Frequenzen erzeugt.

11. Verfahren nach Anspruch 9 oder 10, **dadurch gekennzeichnet, dass** das Schwingungselement Schwingungen im Hörschall erzeugt.

12. Verfahren nach einem der Ansprüche 9 bis 11, **dadurch gekennzeichnet, dass** das Schwingungselement zum Bekämpfen von Polyederschaum Schwingungen von zumindest 16 Hz, vorzugsweise 25 Hz bis 1000 Hz und insbesondere zumindest 50 Hz bis 500 Hz erzeugt.

13. Verfahren nach einem der Ansprüche 9 bis 12, **dadurch gekennzeichnet, dass** das Schwingungselement zum Bekämpfen der Blasendispersion Schwingungen von nicht mehr als 16 kHz, insbesondere nicht mehr als 10 kHz oder nicht mehr als 100 kHz und vorzugsweise nicht mehr als 500 Hz beträgt.

14. Verfahren nach einem der Ansprüche 9 bis 13, **dadurch gekennzeichnet, dass** es sich bei der Vibrationsvorrichtung um einen 1-Modus Schwingbalken handelt.

15. Verfahren nach einem der Ansprüche 9 bis 14, **dadurch gekennzeichnet, dass** ein Fermenter gemäß einem der Ansprüche 1 bis 8 verwendet wird.

**Claims**

1. Fermenter for producing biogas, in which fermenter a fermentation medium can be received up to a predetermined fill level, a vibration device being provided in the fermenter, the vibration device being an oscillating beam or an internal vibrator, **characterized in that** the oscillating element is at a distance of at least 0.5 m from a bottom of the fermenter and/or is in the region of the middle third or the upper third of the height of the fill level in the biogas fermenter.

2. Fermenter according to claim 1, **characterized in that** the oscillating beam is designed to carry out axial oscillations or angular oscillations and/or **in that** the vibration device is designed to generate variable frequencies.

3. Fermenter according to either claim 1 or claim 2, **characterized in that** the oscillations generated are audible sound.

4. Fermenter according to any of claims 1 to 3, **characterized in that** the vibration device comprises a drive and a vibration transmission element.

5. Fermenter according to claim 4, **characterized in that** the drive is arranged outside the biogas fermenter and/or **in that** the drive is a pneumatic drive, an asynchronous motor, an unbalance motor or a piezo oscillator.

6. Fermenter according to any of the preceding claims, **characterized in that** the frequency generated for combating polyhedron foam is at least 16 Hz, preferably 25 Hz to 1000 Hz, and in particular at least 50 Hz to 500 Hz.

7. Fermenter according to any of claims 1 to 6, **characterized in that** the frequency generated for combating the bubble dispersion is no more than 16 kHz, in particular no more than 10 kHz or no more than 100 kHz, and preferably no more than 500 Hz.

8. Fermenter according to any of claims 1 to 7, **characterized in that** the vibration device is a single-mode oscillating beam.

9. Method for reducing the foam production and/or improving degassing in a biogas fermenter, oscillations being transferred to a fermentation medium located in the biogas fermenter by means of an oscillating beam or internal vibrator, **characterized in that** the oscillating element is at a distance of at least 0.5 m from a bottom of the fermenter and/or is arranged in the region of the middle third or the upper third of the height of the fill level in the biogas fermenter.

10. Method according to claim 9, **characterized in that** the oscillating beam is set into axial oscillation or angular oscillation and/or **in that** the oscillating element generates oscillations having variable frequencies.

11. Method according to either claim 9 or claim 10, **characterized in that** the oscillating element generates oscillations in audible sound.

12. Method according to any of claims 9 to 11, **characterized in that** the oscillating element for combating polyhedron foam generates oscillations of at least 16 Hz, preferably 25 Hz to 1000 Hz, and in particular at least 50 Hz to 500 Hz.

13. Method according to any of claims 9 to 12, **characterized in that** the oscillating element for combating the bubble dispersion reaches oscillations of no more than 16 kHz, in particular no more than 10 kHz or no more than 100 kHz, and preferably no more than 500 Hz.

14. Method according to any of claims 9 to 13, **characterized in that** the vibration device is a single-mode oscillating beam.

15. Method according to any of claims 9 to 14, **characterized in that** a fermenter according to any of claims 1 to 8 is used.

**Revendications**

1. Fermenteur pour la production de biogaz, dans lequel un milieu de fermentation peut être amené à un niveau de remplissage prédéterminé, un dispositif de vibration étant prévu dans le fermenteur, le dispositif de vibration étant une barre vibrante ou un vibrateur interne, **caractérisé en ce que** l'élément de vibration se trouve à au moins 0,5 m du fond du fermenteur et/ou se situe dans la région du tiers médian ou du tiers supérieur de la hauteur du niveau de remplissage dans le fermenteur à biogaz.

2. Fermenteur selon la revendication 1, **caractérisé en ce que** la barre vibrante est conçue pour effectuer des vibrations axiales ou angulaires et/ou que le dispositif de vibration est conçu pour générer des fréquences variables.

3. Fermenteur selon la revendication 1 ou 2, **caractérisé en ce que** les vibrations générées sont des sons audibles.

**4.** Fermenteur selon l'une des revendications 1 à 3, **caractérisé en ce que** le dispositif de vibration présente un entraînement et un élément de transmission de vibration.

**5.** Fermenteur selon la revendication 4, **caractérisé en ce que** l'entraînement est disposé à l'extérieur du fermenteur à biogaz et/ou que l'entraînement est un entraînement pneumatique, un moteur asynchrone, un moteur à balourd ou un oscillateur piézoélectrique.

**6.** Fermenteur selon l'une des revendications précédentes, **caractérisé en ce que** la fréquence générée pour lutter contre la mousse de polyèdre est d'au moins 16 Hz, de préférence de 25 Hz à 1000 Hz et en particulier d'au moins 50 Hz à 500 Hz.

**7.** Fermenteur selon l'une des revendications 1 à 6, **caractérisé en ce que** la fréquence générée pour lutter contre la dispersion des bulles est inférieure à 16 kHz, en particulier à 10 kHz ou à 100 kHz et de préférence à 500 Hz.

**8.** Fermenteur selon l'une des revendications 1 à 7, **caractérisé en ce que** le dispositif de vibration est une barre vibrante à 1 mode.

**9.** Procédé pour réduire la production de mousse et/ou améliorer le dégazage dans un fermenteur à biogaz, dans lequel les vibrations sont transférées avec une barre vibrante ou un vibrateur interne dans un milieu de fermentation situé dans le fermenteur à biogaz, **caractérisé en ce que** l'élément de vibration se trouve à au moins 0,5 m du fond du fermenteur et/ou se situe dans la région du tiers médian ou du tiers supérieur de la hauteur du niveau de remplissage dans le fermenteur à biogaz.

**10.** Procédé selon la revendication 9, **caractérisé en ce que** la barre vibrante est réglée en vibrations axiales ou en vibrations angulaires et/ou que l'élément de vibration génère des vibrations à fréquences variables.

**11.** Procédé selon la revendication 9 ou 10, **caractérisé en ce que** l'élément de vibration génère des vibrations sous forme de sons audibles.

**12.** Procédé selon l'une des revendications 9 à 11, **caractérisé en ce que** l'élément de vibration pour lutter contre la mousse de polyèdre génère des vibrations d'au moins 16 Hz, de préférence de 25 Hz à 1000 Hz et en particulier d'au moins 50 Hz à 500 Hz.

**13.** Procédé selon l'une des revendications 9 à 12, **caractérisé en ce que** l'élément de vibration pour lutter contre la dispersion des bulles produit des vibrations inférieures à 16 kHz, en particulier inférieures à 10 kHz ou à 100 kHz et de préférence inférieures à 500 Hz.

**14.** Procédé selon l'une des revendications 9 à 13, **caractérisé en ce que** le dispositif de vibration est une barre vibrante à 1 mode.

**15.** Procédé selon l'une des revendications 9 à 14, **caractérisé en ce qu'**un fermenteur selon l'une des revendications 1 à 8 est utilisé.

FIG. 1

**FIG. 2**

FIG. 3

EP 3 344 744 B1

FIG. 4

FIG. 5a

1

32

42

39

38

43

39

41

31

2

FIG. 5b

EP 3 344 744 B1

FIG. 6

FIG. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 202013000693 U1
- DE 19850822 B4
- DE 2548441 A1
- DD 60022 A1
- US 4334997 A
- DE 440256 A1
- EP 2803729 A2
- EP 1757562 A1
- DE 3943416 A1
- DE 10224172 B4
- GB 1075100 A
- DE 1769953 B
- DE 871438 B
- DE 972899 B
- US 20060172405 A1
- DE 60130714 T2
- DE 4205739 A1
- DE 2013225322 A1
- DE 102013206492 A1
- EP 2769764 A1
- DE 102005041798 A1
- EP 14185121 A2
- DE 202013104292 U1
- EP 14191283 A2

**In der Beschreibung aufgeführte Nicht-Patentliteratur**

- **FOKUSHEFT.** Schaumbildung in Biogasanlagen. *Deutschen Biomasse Forschungszentrums (DBFZ),* 2015
- **KORNEK et al.** Oscillations of soap bubbles. *New Journal of Physics,* vol. 12 (1010), 073031